# EUROPEAN PATENT SPECIFICATION

(11) **EP 1 544 309 B1**
(45) Date of publication and mention of the grant of the patent: **28.02.2007**
(21) Application number: 04029887.9
(22) Date of filing: 16.12.2004
(51) Int. Cl.: C12Q 1/68

(54) **Assay for detecting methylation status by methylation specific primer extension (MSPE)**
Methylierungsstatus-Detektionsassays mittels methylierungsspezifischer Primerextension (MSPE)
Test de détection de l'état de méthylation par extension de primers spécifiques de la méthylation

(30) Priority: 16.12.2003 US 530010 P
(43) Date of publication of application: 22.06.2005
(73) Proprietor: Bayer HealthCare LLC, Tarrytown N.Y. 10591 (US)
(72) Inventor: Li, Zheng, Hayward CA 94544 (US); Harvey, Jeanne, Livermore CA 94550 (US)
(74) Representative: Forstmeyer, Dietmar

(56) References cited:
- WO-A-00/26401
- WO-A-98/56952

### SEQUENCE LISTING

<110> Bayer HealthCare LLC
   Li, Zheng
   Harvey, Jeanne
<120> ASSAY FOR DETECTING METHYLATION STATUS BY METHYLATION SPECIFIC PRIMER EXTENSION (MSPE)
<130> 1657/2042
<150> us 60/530,010
   <151> 2003-12-16
<160> 14
<170> PatentIn version 3.3
<210> 1
   <211> 24
   <212> DNA
   <213> Artificial
<220>
   <223> Primer
<400> 1
   cgaacgctct gaggtcacag cgtc 24
<210> 2
   <211> 22
   <212> DNA
   <213> Artificial
<220>
   <223> Primer
<400> 2
   gtgctccaga ggctgatgcc gc 22
<210> 3
   <211> 340
   <212> DNA
   <213> Homo sapiens
<400> 3
   gaagaaagag gaggggctgg ctggtcacca gagggtgggg cggaccgcgt gcgctcggcg 60
   gctgcggaga gggggagagc aggcagcggg cggcggggag cagcatggag ccggcggcgg 120
   ggagcagcat ggagccttcg gctgactggc tggccacggc cgcggcccgg ggtcgggtag 180
   aggaggtgcg ggcgctgctg gaggcggggg cgctgcccaa cgcaccgaat agttacggtc 240
   ggaggccgat ccaggtgggt agagggtctg cagcgggagc aggggatggc gggcgactct 300
   ggaggacgaa gtttgcaggg gaattggaat caggtagcgc 340
<210> 4
   <211> 340
   <212> DNA
   <213> Artificial
<220>
   <223> promoter sequence after bisulfite treatment
<220>
   <221> modified_base
   <222> (1)..(340)
   <223> Y at positions 41, 46, 48, 52, 56, 59, 65, 87, 91, 94, 112, 115, 118, 139, 157, 161, 163, 168, 174, 189, 193, 205, 211, 221, 226, 236, 240, 247, 274, 290, 294, 307 and 338 denotes for C/T.
<400> 4
   gaagaaagag gaggggttgg ttggttatta gagggtgggg yggatygygt gygttyggyg     60
   gttgyggaga gggggagagt aggtagyggg yggyggggag tagtatggag tyggyggygg    120
   ggagtagtat ggagttttyg gttgattggt tggttayggt ygyggttygg ggtygggtag    180
   aggaggtgyg ggygttgttg gaggygdggg ygttgtttaa ygtatygaat agttayggty    240
   ggaggtygat ttaggtgggt agagggtttg tagygggagt aggggatggy gggygatttt    300
   ggaggaygaa gtttgtaggg gaattggaat taggtagygt                          340
<210> 5
   <211> 20
   <212> DNA
   <213> Artificial
<220>
   <223> primer
<400> 5
   gaagaaagag gaggggttgg 20
<210> 6
   <211> 20
   <212> DNA
   <213> Artificial
<220>
   <223> primer
<400> 6
   ctacaaaccc tctacccacc 20
<210> 7
   <211> 46
   <212> DNA
   <213> Artificial
<220>
   <223> primer
<220>
   <221> misc_feature
   <223> "n" at positions 3, 7, 9, 14, 16 and 20 is modified base
<220>
   <221> misc_feature
   <222> (3).. (3)
   <223> n is a, c, g, or t
<220>
   <221> misc_feature
   <222> (7) ..(7)
   <223> n is a, c, g, or t
<220>
   <221> misc_feature
   <222> (9)..(9)
   <223> n is a, c, g, or t
<220>
   <221> misc_feature
   <222> (14) .. (14)
   <223> n is a, c, g, or t
<220>
   <221> misc_feature
   <222> (16)..(16)
   <223> n is a, c, g, or t
<220>
   <221> misc_feature
   <222> (20). .(20)
   <223> n is a, c, g, or t
<400> 7
   gangctntna cctnanagcn ttcgtttcgg ttgattggtt ggttac 46
<210> 8
   <211> 21
   <212> DNA
   <213> Artificial
<220>
   <223> primer
<400> 8
   gctacaaacc ctctacccac c 21
<210> 9
   <211> 45
   <212> DNA
   <213> Artificial
<220>
   <223> primer
<220>
   <221> misc_feature
   <222> (1)..(45)
   <223> "n" at positions 1, 4, 8, 12, 16 and 20 is modified base.
<400> 9
   ncgncatnag cntctngagn acgtttttgg ttgattggtt ggtta 45
<210> 10
   <211> 24
   <212> DNA
   <213> Artificial
<220>
   <223> primer
<400> 10
   cactacaaac cctctaccca ccta 24
<210> 11
   <211> 24
   <212> DNA
   <213> Artificial
<220>
   <223> primer
<220>
   <221> misc_feature
   <223> "n" at positions 5, 9, 11, 16, 18 and 22 is modified base
<220>
   <221> misc_feature
   <222> (5).. (5)
   <223> n is a, c, g, or t
<220>
   <221> misc_feature
   <222> (9) .. (9)
   <223> n is a, c, g, or t
<220>
   <221> misc_feature
   <222> (11).. (11)
   <223> n is a, c, g, or t
<220>
   <221> misc_feature
   <222> (16).. (16)
   <223> n is a, c, g, or t
<220>
   <221> misc_feature
   <222> (18) .(18)
   <223> n is a, c, g, or t
<220>
   <221> misc_feature
   <222> (22).. (22)
   <223> n is a, c, g, or t
<400> 11
   cgaangctnt naggtnanag cntc 24
<210> 12
   <211> 21
   <212> DNA
   <213> Artificial
<220>
   <223> Primer
<220>
   <221> misc feature
   <223> "n" at position 3, 7, 11, 15 and 19 is modified base
<220>
   <221> misc_feature
   <222> (3) .. (3)
   <223> n is a, c, g, or t -
<220>
   <221> misc_feature
   <222> (7) ..(7)
   <223> n is a, c, g, or t
<220>
   <221> misc_feature
   <222> (11) .. (11)
   <223> n is a, c, g, or t
<220>
   <221> misc_feature
   <222> (15). .(15)
   <223> n is a, c, g, or t
<220>
   <221> misc_feature
   <222> (19).. (19)
   <223> n is a, c, g, or t
<400> 12 21
   gtnctcnaga ngctnatgnc g 21
<210> 13
   <211> 24
   <212> DNA
   <213> Artificial
<220>
   <223> primer
<400> 13 24
   gacgctgtga cctcagagcg ttcg 24
<210> 14
   <211> 22
   <212> DNA
   <213> Artificial
<220>
   <223> primer
<400> 14
   gcggcatcag cctctggagc ac 22

## Description

### Field of the Invention

The present invention generally relates to methods for determining the DNA methylation status of CpG sites in genes. Furthermore, the present invention relates to methods of diagnosing disorder or disease by determining the DNA methylation status of nucleic acids in a subject.

### Background of the Invention

In mammals, DNA methylation usually occurs at cytosines located 5' of guanines, known as CpG dinucleotides. DNA (cytosine-5)-methyltransferase (DNA-Mtase) catalyzes this reaction by adding a methyl group from S-adenosyl-L-methionine to the fifth carbon position of the cytosine. Chiang, PK, et al., "S-adenosylmethionine and methylation," *FASEB J*., 10: 471-480 (1996). Most cytosines within CpG dinucleotides are methylated in the human genome, but some remain unmethylated in specific GC-rich areas. These areas are called CpG islands. Antequera, F. et al., "High levels of *de novo* methylation and altered chromatin structure at CpG islands in cell lines," *Cell,* 62: 503-514 (1990). CpG islands are typically between 0.2 to about 1kb in length and are located upstream of many housekeeping and tissue-specific genes, but may also extend into gene coding regions. Antequera, F. et al., "High levels of *de novo* methylation and altered chromatin structure at CpG islands in cell lines," *Cell*, 62: 503-514 (1990).

DNA methylation is a heritable, reversible, and epigenetic change, it has the potential to alter gene expression, which has profound developmental and genetic consequences. DNA methylation is known to play a role in regulating gene expression during cell development. This epigenetic event frequently is associated with transcriptional silencing of imprinted genes, some repetitive elements and genes on the inactive X chromosome. Li, E. et al, "Role for DNA methylation in genomic imprinting," *Nature*, 366: 362-365 (1993); Singer-Sam, J. and Riggs, AD, X chromosome inactivation and DNA methylation. Jost, J.P. and Saluz, H.P. (eds), *DNA Methylation: molecular Biology and Biological Significance*. Birkhaeuser Verlag, Basel, Switzerland, pp. 358-384 (1993). In neoplastic cells, it has been observed that the normally unmethylated CpG islands can become aberrantly methylated, or hypermethylated. Jones, PA, "DNA methylation errors and cancer," *Cancer Res.,* 56:2463-2467 (1996).

Aberrantly methylated cytosine at CpG dinucleotides is a widespread phenomenon in cancer. Jones, PA and Laird, PW, "Cancer epigenetics comes of age," *Nat. Genet*. 21: 163-167 (1999). As a result of CpG island hypermethylation, chromatin structure in the promoter can be altered, preventing normal interaction with the transcriptional machinery. Baylin, SB, et al. "Alterations in DNA methylation: A fundamental aspect of neoplasia," in *Advances in cancer research* (eds. G.F. Vande Woude and G. Klein), vol. 72: 141-196 (1998), Academic Press, San Diego, CA. When this occurs in genes critical to growth inhibition, the resulting silencing of transcription could promote tumor progression. In addition, promoter CpG island hypermethylation has been shown to be a common mechanism for transcriptional inactivation of classic tumor suppressor genes and genes important for cell cycle regulation, and DNA mismatch repair.

Based on these discoveries, it can be said that methylation of cytosine plays a significant role in control of gene expression, and change of the methylation pattern or status should cause diseases.

### Summary of the Invention

The present invention provides a method for determining DNA methylation state or status at a specific CpG site or sites of interest. The method comprises:
(a) obtaining and/or using DNA from a sample to be analyzed;
(b) contacting the DNA with an agent that modifies unmethylated cytosine to uracil while leaving any 5'-methylated cytosine unchanged;
(c) amplifying the DNA using strand-specific primers;
(d) performing a primer extension reaction using at least one pair of methylation specific primer extension (MSPE) primers that hybridize to the top strand of the amplified DNA, labeled dNTPs, and a DNA polymerase, wherein the 3'-end of the first MSPE primer in the pair comprises a polynucleotide sequence that is specific for the top strand of the methylated DNA, the most 3'-end of the primer hybridizes at the cytosine residue of the CpG site to be analyzed, and the 5'-end of said first MSPE primer comprises a first unique sequence that does not hybridize to any DNA sequences in the sample; wherein the 3'-end of the second MSPE primer in the pair comprises a polynucleotide sequence that is specific for the top strand of the unmethylated DNA sequence, the most 3'-end of the primer hybridizes at the thymine residue which is derived from the cytosine of the CpG site to be analyzed, and the 5'-end of said second MSPE primer comprises a second unique sequence that does not hybridize to any DNA sequences in the sample;
(e) hybridizing the primer extension products from (d) to at least one pair of oligonucleotides, wherein the first oligonucleotide in the pair is complementary to said first unique sequence, and the second oligonucleotide in the pair is complementary to said second unique sequence; and
(f) determining the 5'-methylation status at the cytosine residue of the CpG site by comparing the hybridization intensity of the methylated DNA to the hybridization intensity of the unmethylated DNA in the sample.

In another aspect, the present invention provides a method for determining DNA methylation state or status at a specific CpG site or sites of interest, comprising the steps of:
(a) obtaining and/or using DNA from a sample to be analyzed;
(b) contacting the DNA with an agent that modifies unmethylated cytosine to uracil while leaving any 5'-methylated cytosine unchanged;
(c) amplifying the DNA using strand-specific primers;
(d) performing a primer extension reaction using at least one pair of MSPE primers that hybridize to the bottom strand of the amplified DNA, labeled dNTPs, and a DNA polymerase, wherein the 3'-end of the first MSPE primer in the pair comprises a polynucleotide sequence that is specific for the bottom strand of the methylated DNA, the most 3'-end of the primer hybridizes at the guanine residue complementary to the cytosine of the CpG site on the top strand, and the 5'-end of said first MSPE primer comprises a first unique sequence that does not hybridize to any DNA sequences in the sample; wherein the 3'-end of the second MSPE primer in the pair comprises a polynucleotide sequence that is specific for the bottom strand of the unmethylated DNA sequence, the most 3'-end of the primer hybridizes at the adenine residue which is derived from the cytosine of the CpG site on the top strand, and the 5'-end of said second MSPE primer comprises a second unique sequence that does not hybridize to any DNA sequences in the sample;
(e) hybridizing the primer extension products from (d) to at least one pair of oligonucleotides, wherein the first oligonucleotide in the pair is complementary to said first unique sequence, and the second oligonucleotide in the pair is complementary to said second unique sequence.
(f) determining the 5'-methylation status at the cytosine residue of the CpG site by comparing the hybridization intensity of the methylated DNA to the hybridization intensity of the unmethylated DNA in the sample.

In another aspect, the present invention provides a method for determining DNA methylation state or status at a specific CpG site or sites of interest, comprising the steps of:
(a) obtaining and/or using DNA from a sample to be analyzed;
(b) contacting the DNA with an agent that modifies unmethylated cytosine to uracil while leaving any 5'-methylated cytosine unchanged;
(c) amplifying the DNA using strand-specific primers;
(d) performing a primer extension reaction using at least one pair of methylation specific primer extension (MSPE) primers that hybridize to the top strand of the amplified DNA, dNTPs, labeled ddCTP and a DNA polymerase, wherein the 3'-end of the first MSPE primer in the pair comprises a polynucleotide sequence that is specific for the top strand of the methylated DNA, the most 3'-end of the primer hybridizes at the cytosine residue of the CpG site to be analyzed, and the 5'-end of said first MSPE primer comprises a first unique sequence that does not hybridize to any DNA sequences in the sample; wherein the 3'-end of the second MSPE primer in the pair comprises a polynucleotide sequence that is specific for the top strand of the unmethylated DNA sequence, the most 3'-end of the primer hybridizes at the thymine residue which is derived from the cytosine of the CpG site to be analyzed, and the 5'-end of said second MSPE primer comprises a second unique sequence that does not hybridize to any DNA sequences in the sample;
(e) hybridizing the primer extension products from (d) to at least one pair of oligonucleotides, wherein the first oligonucleotide in the pair is complementary to said first unique sequence, and the second oligonucleotide in the pair is complementary to said second unique sequence; and
(f) determining the 5'-methylation status at the cytosine residue of the CpG site by comparing the hybridization intensity of the methylated DNA to the hybridization intensity of the unmethylated DNA in the sample.

Alternatively, in another aspect, the present invention provides a method for determining DNA methylation state or status at a specific CpG site or sites of interest, comprising the steps of:
(a) obtaining and/or using DNA from a sample to be analyzed;
(b) contacting the DNA with an agent that modifies unmethylated cytosine to uracil while leaving any 5'-methylated cytosine unchanged;
(c) amplifying the DNA using strand-specific primers;
(d) performing a primer extension reaction using at least one pair of methylation specific primer extension (MSPE) primers that hybridize to the top strand of the amplified DNA, mixture of labeled dNTPs and ddNTPs, mixture of unlabeled dNTPs and ddNTPs, and a DNA polymerase, wherein the 3'-end of the first MSPE primer in the pair comprises a polynucleotide sequence that is specific for the top strand of the methylated DNA, the most 3'-end of the primer hybridizes at the cytosine residue of the CpG site to be analyzed, and the 5'-end of said first MSPE primer comprises a first unique sequence that does not hybridize to any DNA sequences in the sample; wherein the 3'-end of the second MSPE primer in the pair comprises a polynucleotide sequence that is specific for the top strand of the unmethylated DNA sequence, the most 3'-end of the primer hybridizes at the thymine residue which is derived from the cytosine of the CpG site to be analyzed, and the 5'-end of said second MSPE primer comprises a second unique sequence that does not hybridize to any DNA sequences in the sample;
(e) hybridizing the primer extension products from (d) to at least one pair of oligonucleotides, wherein the first oligonucleotide in the pair is complementary to said first unique sequence, and the second oligonucleotide in the pair is complementary to said second unique sequence; and
(f) determining the 5'-methylation status at the cytosine residue of the CpG site by comparing the hybridization intensity of the methylated DNA to the hybridization intensity of the unmethylated DNA in the sample.

In another aspect, the present invention provides a method for determining DNA methylation state or status at a specific CpG site or sites of interest, comprising the steps of:
(a) obtaining and/or using DNA from a sample to be analyzed;
(b) contacting the DNA with an agent that modifies unmethylated cytosine to uracil while leaving any 5'-methylated cytosine unchanged;
(c) amplifying the DNA using strand-specific primers;
(d) performing a primer extension reaction using at least one pair of MSPE primers that hybridize to the bottom strand of the amplified DNA, mixture of labeled dNTPs and ddNTPs, mixture of unlabeled dNTPs and ddNTPs, and a DNA polymerase, wherein the 3'-end of the first MSPE primer in the pair comprises a polynucleotide sequence that is specific for the bottom strand of the methylated DNA, the most 3'-end of the primer hybridizes at the guanine residue complementary to the cytosine of the CpG site on the top strand, and the 5'-end of said first MSPE primer comprises a first unique sequence that does not hybridize to any DNA sequences in the sample; wherein the 3'-end of the second MSPE primer in the pair comprises a polynucleotide sequence that is specific for the bottom strand of the unmethylated DNA sequence, the most 3'-end of the primer hybridizes at the adenine residue which is derived from the cytosine of the CpG site on the top strand, and the 5'-end of said second MSPE primer comprises a second unique sequence that does not hybridize to any DNA sequences in the sample;
(e) hybridizing the primer extension products from (d) to at least one pair of oligonucleotides, wherein the first oligonucleotide in the pair is complementary to said first unique sequence, and the second oligonucleotide in the pair is complementary to said second unique sequence.
(f) determining the 5'-methylation status at the cytosine residue of the CpG site by comparing the hybridization intensity of the methylated DNA to the hybridization intensity of the unmethylated DNA in the sample.

Alternatively, in another aspect, the present invention provides a method for determining DNA methylation state or status at a specific CpG site or sites of interest, comprising the steps of:
(a) obtaining and/or using a DNA from a sample to be analyzed;
(b) contacting the DNA with an agent that modifies unmethylated cytosine to uracil while leaving any 5'-methylated cytosine unchanged;
(c) amplifying the DNA using strand-specific primers;
(d) performing a primer extension reaction using at least a pair of MSPE primers that hybridize to the top strand of the amplified DNA, at least one labeled reverse primer, dNTPs, and a DNA polymerase, wherein the 5'-end of the first MSPE primer in the pair comprises a first unique sequence that does not hybridize to any DNA sequences in the sample, and the 3'-end of the first MSPE primer comprises a polynucleotide sequence that is specific for the top strand of the methylated DNA, the most 3'-end of the primer hybridizes at the cytosine residue of the CpG site to be analyzed; wherein the 5'-end of the second MSPE primer in the pair comprises a second unique sequence that does not hybridize to any DNA sequences in the sample, and the 3'-end of the second MSPE primer in the pair comprises a polynucleotide sequence that is specific for the top strand of the unmethylated DNA, the most 3'-end of the primer hybridizes at the thymine residue which is derived from the cytosine of the CpG site to be analyzed;
(e) hybridizing the primer extension products from (d) to at least a pair of oligonucleotides, wherein the first oligonucleotide in the pair is the same as the first unique sequence and the second oligonucleotide in the pair is the same as the second unique sequence; and
(f) determining the 5'-methylation status at the cytosine residue of the CpG site by comparing the hybridization intensity of the methylated DNA to the hybridization intensity of the unmethylated DNA in the sample.

In another aspect, the present invention provides a method for determining DNA methylation state or status at a specific CpG site or sites of interest, comprising the steps of:
(a) obtaining and/or using a DNA from a sample to be analyzed;
(b) contacting the DNA with an agent that modifies unmethylated cytosine to uracil while leaving any 5'-methylated cytosine unchanged;
(c) amplifying the DNA using strand-specific primers;
(d) performing a primer extension reaction using at least a pair of MSPE primers that hybridize to the bottom strand of the amplified DNA, at least one labeled reverse primer, dNTPs, and a DNA polymerase, wherein the 5'-end of the first MSPE primer in the pair comprises a first unique sequence that does not hybridize to any DNA sequences in the sample, and the 3'-end of the first MSPE primer comprises a polynucleotide sequence that is specific for the bottom strand of the methylated DNA, the most 3'-end of the primer hybridizes at the guanine residue complementary to the cytosine of the CpG site on the top strand; wherein the 5'-end of the second MSPE primer in the pair comprises a second unique sequence that does not hybridize to any DNA sequences in the sample, and the 3'-end of the second MSPE primer in the pair comprises a polynucleotide sequence that is specific for the bottom strand of the unmethylated DNA, the most 3'-end of the primer hybridizes at the adenine residue which is derived from the cytosine of the CpG site on the top strand;
(e) hybridizing the primer extension products from (d) to at least a pair of oligonucleotides, wherein the first oligonucleotide in the pair is the same as the first unique sequence and the second oligonucleotide in the pair is the same as the second unique sequence; and
(f) determining the 5'-methylation status at the cytosine residue of the CpG site by comparing the hybridization intensity of the methylated DNA to the hybridization intensity of the unmethylated DNA in the sample.

According to the present invention, it is preferred that the DNA in step (a) is genomic DNA.

According to the present invention, it is preferred that the agent used to convert the unmethylated cytosine into uracil is a bisulfite compound. Preferably, the agent used for modifying unmethylated cytosine is sodium bisulfite.

According to the present invention, it is preferred that PCR is used to amplify the chemically treated nucleic acids. In one preferred embodiment, the PCR amplification is conducted with at least a pair of PCR primers, and the PCR primers should recognize both the methylated and unmethylated DNA templates. The PCR primers may comprise a sequence that hybridizes under stringent conditions to at least about 7, preferably about 12, preferably about 15, more preferably about 25, 50, 75, 100, or more nucleotides, ideally about 17 to 40 nucleotides. In another preferred embodiment, multiple pairs of PCR primers recognizing multiple CpG sites are used, and multiplex PCR is performed to concurrently amplify the DNA template.

According to the present invention, MSPE reactions are performed for analysis of methylation levels of one or more CpG sites on one or more nucleic acid molecules. In a preferred embodiment, several single or multiplexed PCR reactions can be pooled while conducting the MSPE reactions. In one embodiment, MSPE primers used in the primer extension reaction are designed to hybridize to sequences that originally contain CpG dinucleotides.

For analysis of one defined CpG site, preferably at least two classes of MSPE primers are included in the extension reaction. One class of MSPE primer is methylation specific and anneals to the methylated CpG sites and the other class of MSPE primer is specific for non-methylated CpG sites and anneals to the unmethylated CpG sites of the amplified nucleic acids. Each class of MSPE primer consists of two moieties. The 3' end portion anneals to defined CpG site(s) on the amplified nucleic acids, and the 5' end portion anneals to a unique/adapter sequence that does not anneal to any of the amplified nucleic acids.

In one embodiment, the primer extension reaction is conducted by using MSPE primers that anneal to the top strand of the amplified DNA and by using labeled dNTPs, wherein dATP, dTTP, and dCTP may be labeled. In another embodiment, the primer extension reaction is conducted by using MSPE primers that anneal to the top strand of the amplified DNA and by using labeled ddCTP. In another embodiment, the primer extension reaction is conducted by using MSPE primers that anneal to the top strand of the amplified DNA and by using mixture of labeled dNTPs and ddNTPs, and mixture of unlabeled dNTPs and ddNTPs. In another embodiment, the primer extension reaction is conducted by using MSPE primers that anneal to the bottom strand of the template DNA and by using the labeled dNTPs, wherein dATP, dTTP, and dGTP may be labeled. In another embodiment, the primer extension reaction is conducted by using MSPE primers that anneal to the bottom strand of the amplified DNA and by using mixture of labeled dNTPs and ddNTPs, and mixture of unlabeled dNTPs and ddNTPs. In another embodiment, the primer extension reaction is conducted by using labeled reverse primers that anneal to the top strand of the amplified DNA using the unlabeled dNTPs. In another embodiment, the primer extension reaction is conducted by using labeled reverse primers that anneal to the bottom strand of the amplified DNA using the unlabeled dNTPs.

The dNTPs, ddNTPs or reverse primers that are incorporated into the extension products may be labeled with a detectable label. Detectable labels are well known in the art. Any type of detectable label may be used. In a preferred embodiment, the label is a radioisotope. In another preferred embodiment, the label is a fluorescent agent. In another preferred embodiment, the label is a binding moiety such as biotin.

The detectable label may be either a primary label that can be directly detected, or a secondary label that can be indirectly detected.

In one aspect, the present invention provides a method for determining and quantifying relative methylation levels at one or more CpG sites by analyzing the signal intensity of the MSPE reaction product containing one or more CpG sites. In a preferred embodiment, large numbers of CpG sites can be determined for their relative methylation level simultaneously, e.g., the determination and quantification of relative methylation levels at one or more CpG sites can be performed in a high throughput manner or on a microarray.

Another aspect of the present invention also provides a kit for detection of relative methylation levels at one or more CpG sites. The kit may include (1) an agent that modifies unmethylated cytosine nucleotides, (2) primers for amplification of the nucleic acid molecule, (3) MSPE primers as defined in claim 1 for the methylated CpG-containing nucleic acid (either labeled or unlabeled), (4) MSPE primers as defined in claim 1 for the unmethylated CpG-containing nucleic acid (either labeled or unlabeled), (5) labeled and unlabeled dNTPs, (6) labeled and unlabeled ddNTPs, and (7) labeled or unlabeled reverse primers. The kit may further include nucleic acid amplification buffer and reagents for MSPE reactions. Preferably, the agent that modifies unmethylated cytosine is a bisulfite compound.

Another aspect of the present invention also provides a method of using the present method and/or kit to determine a predisposition to a disease or disorder, or to diagnose and/or prognose a disease or disorder, or to monitor therapeutic response of a drug or compound, in a subject comprising determining the relative methylation levels at one or more CpG sites on a nucleic acid molecule, and comparing the relative methylation level in the subject to the relative methylation level of said nucleic acid molecule from a control subject (or standard) not having a predisposition to the disease or disorder, wherein a significant difference in the relative methylation level is indicative of the predisposition to the disease or disorder in the subject.

### Brief Description of the Figures

Figure 1 shows fluorescence readout of both methylated and unmethylated DNA template.
Figure 2. shows the ratio of expected methylation over observed.
Figure 3. shows the predictive power of the assay (calculated ratio vs. expected ratio).
Figure 4. shows a schematic description of the invention.

### Detailed Description of the Invention

### I General

The present invention relates to methods for determining the DNA methylation state or status in samples. In general, the first step of the methods includes chemically modifying the CpG sites, converting the non-methylated cytosines into uracil, leaving the 5'-methylated cytosine unmodified. The chemically treated DNA may then be amplified by conventional molecular biology techniques including PCR amplification. The methylation state or status in the amplified DNA products may then be analyzed by primer extension reaction by using tagged primers or dNTPs or ddNTPs. Another aspect of the present invention relates to the clinical applications of methylation state or status as a disease marker or as basis for treatments. Particularly, abnormal methylation at the CpG sites inactivates gene expressions. Therefore, the relative methylation levels at one or more particular CpG sites as compared to the unmethylation levels at the same sites serves as a diagnostic, prognostic or therapeutic tool.

### Definitions

As used herein, the term "methylation" refers to the covalent attachment of a methyl group at the C5-position of the nucleotide base cytosine within the CpG dinucleotides of gene regulatory region. The term "methylation state" or "methylation status" refers to the presence or absence of 5-methyl-cytosine ("5-mCyt") at one or a plurality of CpG dinucleotides within a DNA sequence. As used herein, the terms "methylation status" and "methylation state" are used interchangeably. A methylation site is a sequence of contiguous linked nucleotides that is recognized and methylated by a sequence-specific methylase. A methylase is an enzyme that methylates (i. e., covalently attaches a methyl group) one or more nucleotides at a methylation site.
As used here, the term "CpG islands" are short DNA sequences rich in CpG dinucleotide and can be found in the 5' region of about one-half of all human genes. The term "CpG site" refers to the CpG dinucleotide within the CpG islands. CpG islands are typically, but not always, between about 0.2 to about 1 kb in length.

As used herein, the term "sample" as used in its broadest sense, refers to any plant, animal or viral material containing DNA or RNA, such as, for example, tissue or fluid isolated from an individual (including without limitation plasma, serum, cerebrospinal fluid, lymph, tears, saliva and tissue sections) or from in vitro cell culture constituents, as well as samples from the environment. The term "sample" also refers to "a biological sample." As used herein, the term "a biological sample" refers to a whole organism or a subset of its tissues, cells or component parts (e.g. body fluids, including but not limited to blood, mucus, lymphatic fluid, synovial fluid, cerebrospinal fluid, saliva, amniotic fluid, amniotic cord blood, urine, vaginal fluid and semen). "A biological sample" further refers to a homogenate, lysate or extract prepared from a whole organism or a subset of its tissues, cells or component parts, or a fraction or portion thereof, including but not limited to, for example, plasma, serum, spinal fluid, lymph fluid, the external sections of the skin, respiratory, intestinal, and genitourinary tracts, tears, saliva, milk, blood cells, tumors, organs. Most often, the sample has been removed from an animal, but the term "biological sample" can also refer to cells or tissue analyzed *in vivo*, i.e., without removal from animal. Typically, a "biological sample" will contain cells from the animal, but the term can also refer to non-cellular biological material, such as non-cellular fractions of blood, saliva, or urine, that can be used to measure the cancer-associated polynucleotide or polypeptides levels. "A biological sample" further refers to a medium, such as a nutrient broth or gel in which an organism has been propagated, which contains cellular components, such as proteins or nucleic acid molecules.

As used herein, the term "agent" refers to any compound that has the property to modify unmethylated cytosine to another nucleotide while leaving any 5'-methylated cytosine unchanged. The modification will distinguish the unmethylated from the methylated cytosine. Preferably, the agent modifies unmethylated cytosine to uracil. Preferably, the agent used for modifying unmethylated cytosine is sodium bisulfite. However, other agents that similarly modify unmethylated cytosine, but not methylated cytosine can also be used in the present invention.

As used herein, the term "primer" refers to a polynucleotide which, whether purified from a nucleic acid restriction digest or produced synthetically, is capable of acting as a point of initiation of nucleic acid synthesis when placed under conditions in which synthesis of a primer extension product which is complementary to a nucleic acid strand is induced, *i*.*e*., in the presence of nucleotides and an agent for polymerization such as DNA polymerase, reverse transcriptase or the like, and at a suitable temperature and pH. The primer is preferably single stranded for maximum efficiency, but may alternatively be double stranded. If double stranded, the primer is first treated to separate its strands before being used to prepare extension products. Preferably, the primer is a polydeoxyribonucleotide. The primer must be sufficiently long to prime the synthesis of extension products in the presence of the agents for polymerization. The exact lengths of the primers will depend on many factors, including temperature and the source of primer. For example, depending on the complexity of the target sequence, a polynucleotide primer typically contains 15 to 25 or more nucleotides, although it can contain fewer nucleotides. Short primer molecules generally require cooler temperatures to form sufficiently stable hybrid complexes with template.

As used herein, the term "a MSPE primer" refers to an oligonucleotide that hybridizes to the CpG site to be assayed. "A MSPE primer" contains two moieties. The 5' end of the MSPE primer contains a unique sequence that does not hybridize to any DNA in the sample to be assayed, and the 3' end of the MSPE primer contains a sequence complementary to a region containing CpG sites to be assayed.

As used herein, the term "unique sequence", also referred to as "zip code" or "adapter sequence," is a sequence, generally exogeneous to the target sequences, e.g. artificial. In a preferred embodiment, the adapter sequence is designed to be substantially complementary (and preferably perfectly complementary) to a capture probe of a detection microbead or microarray. Preferably, the capture probe is immobilized to a solid support that can include microspheres or planar substrates such as plastic or glass slides as described herein for array supports. One nonlimiting example is 3' amine modified oligos: CP32: 5' CGAACGCTCTGAGGTCACAGCGTC 3' (SEQ ID NO. 1), CP33: 5' GTGCTCCAGAGGCTGATGCCGC 3' (SEQ ID NO. 2), CE32: 5' GACGCTGTGACCTCAGAGCGTTCG 3' (SEQ ID NO. 13), and CE33: 5' GCGGCATCAGCCTCTGGAGCAC 3' (SEQ NO. 14).

As used herein, the term "amplifying" refers to the process of synthesizing nucleic acid molecules that are complementary to one or both strands of a template nucleic acid. Amplifying a nucleic acid molecule typically includes denaturing the template nucleic acid, annealing primers to the template nucleic acid at a temperature that is below the melting temperatures of the primers, and enzymatically elongating from the primers to generate an amplification product. The denaturing, annealing and elongating steps each can be performed once. Generally, however, the denaturing, annealing and elongating steps are performed multiple times such that the amount of amplification product is increasing, often times exponentially, although exponential amplification is not required by the present methods. Amplification typically requires the presence of deoxyribonucleoside triphosphates, a DNA polymerase enzyme and an appropriate buffer and/or co-factors for optimal activity of the polymerase enzyme. The term "amplification product" refers to the nucleic acid sequences, which are produced from the amplifying process as defined herein.

As used herein, the term "label" refers to any atom or molecule that can be used to provide a detectable (preferably quantifiable) effect, and that can be attached to a nucleic acid or protein. Labels include but are not limited to dyes and radiolabels such as ³²P; binding moieties such as biotin; haptens such as digoxgenin; luminogenic, phosphorescent or fluorogenic moieties; and fluorescent dyes alone or in combination with moieties that can suppress or shift emission spectra by fluorescence resonance energy transfer (FRET). Labels may provide signals detectable by fluorescence, radioactivity, colorimetry, gravimetry, X-ray diffraction or absorption, magnetism, enzymatic activity, and the like. A label may be a charged moiety (positive or negative charge) or alternatively, may be charge neutral. Labels can include or consist of nucleic acid or protein sequence, so long as the sequence comprising the label is detectable. The term "labeled dNTPs" refers to the dNTPs that are modified by the attached labels. The term "labeled ddNTPs" refers to the ddNTPs that are modified by the attached labels.

As used herein, the term "hybridizes" refers to a process in which a nucleic acid strand anneals to and forms a stable duplex, either a homoduplex or a heteroduplex, under normal hybridization conditions with a second complementary nucleic acid strand, and does not form a stable duplex with unrelated nucleic acid molecules under the same normal hybridization conditions. The formation of a duplex is accomplished by annealing two complementary nucleic acid strands in a hybridization reaction. The hybridization reaction can be made to be highly specific by adjustment of the hybridization conditions (often referred to as hybridization stringency) under which the hybridization reaction takes place, such that hybridization between two nucleic acid strands will not form a stable duplex, *e*.*g*., a duplex that retains a region of double-strandedness under normal stringency conditions, unless the two nucleic acid strands contain a certain number of nucleotides in specific sequences which are substantially or completely complementary. "Normal hybridization or normal stringency conditions" are readily determined for any given hybridization reaction. See, for example, Ausubel *et al*., Current Protocols in Molecular Biology, John Wiley & Sons, Inc., New York, or Sambrook *et al*., Molecular Cloning: A Laboratory Manual, Cold Spring Harbor Laboratory Press. As used herein, the term "hybridizing" or "hybridization" refers to any process by which a strand of nucleic acid binds with a complementary strand through base pairing.

As used herein, "a DNA polymerase" refers to an enzyme that catalyzes the polymerization of deoxynucleotides. Generally, the enzyme will initiate synthesis at the 3'-end of the primer annealed to a DNA template sequence, and will proceed in the 5'-direction along the template. Known DNA polymerases include, for example, *Pyrococcus furiosus* (Pfu) DNA polymerase, *E*. *coli* DNA polymerase I, T7 DNA polymerase, *Thermus thermophilus* (Tth) DNA polymerase, *Bacillus stearothermophilus* DNA polymerase, *Thermococcus litoralis* (Tli) DNA polymerase (also referred to as Vent DNA polymerase), *Thermotoga maritima* (UlTma) DNA polymerase, *Thermus aquaticus* (Taq) DNA polymerase, and *Pyrococcus GB-D* (PGB-D) DNA polymerase. The polymerase activity of any of the above enzyme can be defined by means well known in the art. One unit of DNA polymerase activity, according to the subject invention, is defined as the amount of enzyme, which catalyzes the incorporation of 10 nmoles of total dNTPs into polymeric form ending in 30 minutes at 72°C. Preferred thermostable DNA polymerases that may be used in the invention include Taq, Tne, Tma, Pfu, Tfl, Tth, Stoffel fragment, VENT® and DEEPVENT® DNA polymerases, and mutants, variants and derivatives thereof. See U.S. Pat. No. 5,436,149; U.S. Pat. No. 4,889,818; U.S. Pat. No. 4,965, 188; U.S. Pat. No. 5,079,352; U.S. Pat. No. 5,614,365; U.S. Pat. No. 5, 374,553; U.S. Pat. No. 5,270,179; U.S. Pat. No. 5,047,342; U.S. Pat. No. 5,512,462; U.S. Pat. No. 6,015,668; U.S. Pat. No. 5,939,301; U.S. Pat. No. 5,948,614; U.S. Pat. No. 5,912,155; WO 97/09451; WO 98/35060; WO 92/06188; WO 92/06200; WO 96/10640; Barnes, W. M., *Gene* 112:29-35 (1992); Lawyer, F. C., et al., *PCR Meth. Appl*. 2:275-287 (1993); Flaman, J.-M, et al., *Nucl. Acids Res*. 22(15) :3259-3260 (1994).

As used herein, "detecting" refers to the identification of the presence or absence of a molecule in a sample. Where the molecule to be detected is a ligand, the step of detecting can be performed by binding the ligand with an antibody that is detectably labeled. A detectable label is a molecule which is capable of generating, either independently, or in response to a stimulus, an observable signal. A detectable label can be, but is not limited to a fluorescent label, a chromogenic label, a luminescent label, or a radioactive label. Methods for "detecting" a label include quantitative and qualitative methods adapted for standard or confocal microscopy, FACS analysis, and those adapted for high throughput methods involving multiwell plates, arrays or microarrays. One of skill in the art can select appropriate filter sets and excitation energy sources for the detection of fluorescent emission from a given fluorescent ligand or dye. "Detecting" as used herein can also include the use of multiple antibodies to a ligand to be detected, wherein the multiple antibodies bind to different epitopes on the ligand to be detected. Antibodies used in this manner can employ two or more detectable labels, and can include, for example a FRET pair. A polypeptide molecule is "detected" according to the present invention when the level of detectable signal is at all greater than the background level of the detectable label, or where the level of measured nucleic acid is at all greater than the level measured in a control sample.

As used herein, "detecting" also refers to detecting the presence of a target nucleic acid molecule (e.g., a nucleic acid molecule of interest) refers to a process wherein the signal generated by a directly or indirectly labeled probe nucleic acid molecule (capable of hybridizing to a target in a sample) is measured or observed. Thus, detection of the probe nucleic acid is directly indicative of the presence, and thus the detection, of a target nucleic acid, such as a sequence encoding a marker gene. For example, if the detectable label is a fluorescent label, the target nucleic acid is "detected" by observing or measuring the light emitted by the fluorescent label on the probe nucleic acid when it is excited by the appropriate wavelength, or if the detectable label is a fluorescence/quencher pair, the target nucleic acid is "detected" by observing or measuring the light emitted upon association or dissociation of the fluorescence/quencher pair present on the probe nucleic acid, wherein detection of the probe nucleic acid indicates detection of the target nucleic acid. If the detectable label is a radioactive label, the target nucleic acid, following hybridization with a radioactively labeled probe is "detected" by, for example, autoradiography. Methods and techniques for "detecting" fluorescent, radioactive, and other chemical labels may be found in Ausubel et al. (1995, *Short Protocols in Molecular Biology*, 3^{rd} Ed. John Wiley and Sons, Inc.). Alternatively, a nucleic acid may be "indirectly detected" wherein a moiety is attached to a probe nucleic acid which will hybridize with the target, such as an enzyme activity, allowing detection in the presence of an appropriate substrate, or a specific antigen or other marker allowing detection by addition of an antibody or other specific indicator. Alternatively, a target nucleic acid molecule can be detected by amplifying a nucleic acid sample prepared from a patient clinical sample, using oligonucleotide primers, which are specifically designed to hybridize with a portion of the target nucleic acid sequence. Quantitative amplification methods, such as, but not limited to TaqMan, may also be used to "detect" a target nucleic acid according to the invention. A nucleic acid molecule is "detected" as used herein where the level of nucleic acid measured (such as by quantitative PCR), or the level of detectable signal provided by the detectable label is at all above the background level.

As used herein, "detecting" further refers to detecting methylation state or status on a specific CpG site of a target nucleic acid molecule that are indicative of a disease condition in a cell or tissue. The methylation state or status on a specific CpG site of a target nucleic acid molecule can provide useful information for diagnosis, disease monitoring, and therapeutic approaches. Various methods known in the art may be used for determining the methylation status of a specific CpG dinucleotides. Such methods include but not limited to, restriction landmark genomic scanning, see Kawai et al., "Comparison of DNA methylation patterns among mouse cell lines by restriction landmark genomic scanning," *Mol. Cell Biol.* 14(11): 7421-7427 (1994); methylated CpG island amplification, see Toyota et al., "Identification of differentially methylated sequences in colorectal cancer by methylated CpG island amplification," *Cancer Res.,* 59: 2307-2312 (1999), see also WO00/26401A1; differential methylation hybridization, see Huang et al., "Methylation profiling of CpG islands in human breast cancer cells," *Hum. Mol. Genet.,* 8: 459-470 (1999); methylation-specific PCR (MSP), see Herman et al., "Methylation-specific PCR: a novel PCR assay for methylation status of CpG islands," *PNAS USA* 93: 9821-9826 (1992), see also U.S. Patent No. 5,786,146; methylation-sensitive single nucleotide primer extension (Ms-SnuPE), see U.S. Pat. No. 6,251,594; combined bisulfite restriction analysis (COBRA), see Xiong and Laird, "COBRA: a sensitive and quantitative DNA methylation assay," *Nucleic Acids Research*, 25(12): 2532-2534 (1997); bisulfite genomic sequencing, see Frommer et al., "A genomic sequencing protocol that yields a positive display of 5-methycytosine residues in individual DNA strands," *PNAS USA*, 89: 1827-1831 (1992); and methylation-specific primer extension (MSPE), etc.

As used herein, "detecting" refers further to the early detection, or prognoses, or therapeutic response of disease or disorder in a patient. "Detecting" as used herein further refers to the detection of disease recurrence in an individual, using the same detection criteria as indicated above. "Detecting" as used herein still further refers to the measuring of a change in the degree of disease or disorder before and/or after treatment with a therapeutic compound. In this case, a change in the degree of colorectal cancer in response to a therapeutic compound refers to an increase or decrease in the methylation levels of CpG sites by at least 10% in response to the presence of a therapeutic compound relative to the expression level in the absence of the therapeutic compound.

As used herein, the term "nucleic acid" refers to polynucleotides such as deoxyribonucleic acid (DNA), and, where appropriate, ribonucleic acid (RNA). The term should also be understood to include, as equivalents, analogs of either RNA or DNA made from nucleotide analogs, and, as applicable to the embodiment being described, single (sense or antisense) and double-stranded polynucleotides. ESTs, chromosomes, cDNAs, mRNAs, and rRNAs are representative examples of molecules that may be referred to as nucleic acids.

As used herein, the term "cancerous cell" or "cancer cell", used either in the singular or plural form, refers to cells that have undergone a malignant transformation that makes them pathological to the host organism. Malignant transformation is a single- or multi-step process, which involves in part an alteration in the genetic makeup of the cell and/or the gene expression profile. Malignant transformation may occur either spontaneously, or via an event or combination of events such as drug or chemical treatment, radiation, fusion with other cells, viral infection, or activation or inactivation of particular genes. Malignant transformation may occur *in vivo* or *in vitro*, and can if necessary be experimentally induced. Malignant cells may be found within the well-defined tumor mass or may have metastasized to other physical locations. A feature of cancer cells is the tendency to grow in a manner that is uncontrollable by the host, but the pathology associated with a particular cancer cell may take any form. Primary cancer cells (that is, cells obtained from near the site of malignant transformation) can be readily distinguished from non-cancerous cells by well-established pathology techniques, particularly histological examination. The definition of a cancer cell, as used herein, includes not only a primary cancer cell, but any cell derived from a cancer cell ancestor. This includes metastasized cancer cells, and in vitro cultures and cell lines derived from cancer cells.

As used herein, the term "oligonucleotide" refers to a molecule comprising two or more deoxyribonucleotides or ribonucleotides, preferably at least 5 nucleotides, more preferably at least about 10-15 nucleotides and more preferably at least about 15 to 30 or more nucleotides. The exact size will depend on many factors, which in turn depend on the ultimate function or use of the oligonucleotide. The oligonucleotide may be generated in any manner, including chemical synthesis, DNA replication, reverse transcription, PCR, or a combination thereof.

As used herein, the term "isolating" refers to a process in which the material is removed from its original environment (e. g., the natural environment if it is naturally occurring). For example, a naturaly-occurring polynucleotide or polypeptide present in a living animal is not isolated, but the same polynucleotide or DNA or polypeptide, separated from some or all of the coexisting materials in the natural system, is isolated. Such polynucleotide could be part of a vector and/or such polynucleotide or polypeptide could be part of a composition, and still be isolated in that the vector or composition is not part of its natural environment. For example, a naturaly-occurring polynucleotide present in a living animal is not isolated, but the same polynucleotide, separated from some or all of the coexisting materials in the natural system, is isolated. Specifically excluded from the definition of "isolated" are: naturally- occurring chromosomes (such as chromosome spreads), artificial chromosome libraries, genomic libraries, and cDNA libraries that exist either as an in vitro nucleic acid preparation or as a transfected/transformed host cell preparation, wherein the host cells are either an in vitro heterogeneous preparation or plated as a heterogeneous population of single colonies. Also specifically excluded are the above libraries wherein a specified polynucleotide makes up less than 5% of the number of nucleic acid inserts in the vector molecules. Further specifically excluded are whole cell genomic DNA or whole cell RNA preparations (including said whole cell preparations, which are mechanically sheared or enzymatically digested). Further specifically excluded are the above whole cell preparations as either an in vitro preparation or as a heterogeneous mixture separated by electrophoresis (including blot transfers of the same) wherein the polynucleotide of the invention has not further been separated from the heterologous polynucleotides in the electrophoresis medium (e.g., further separating by excising a single band from a heterogeneous band population in an agarose gel or nylon blot).

The present invention provides a method for determining a DNA methylation state or status at a CpG site within CpG islands, comprising the steps of:
(a) obtaining and/or using DNA from a sample to be analyzed;
(b) contacting the DNA with an agent that modifies unmethylated cytosine to uracil while leaving any 5'-methylated cytosine unchanged;
(c) amplifying the DNA using strand-specific primers;
(d) performing a primer extension reaction using at least one pair of methylation specific primer extension (MSPE) primers that hybridize to the top strand of the amplified DNA, labeled dNTPs, and a DNA polymerase, wherein the 3'-end of the first MSPE primer in the pair comprises a polynucleotide sequence that is specific for the top strand of the methylated DNA, the most 3'-end of the primer hybridizes at the cytosine residue of the CpG site to be analyzed, and the 5'-end of said first MSPE primer comprises a first unique sequence that does not hybridize to any DNA sequences in the sample; wherein the 3'-end of the second MSPE primer in the pair comprises a polynucleotide sequence that is specific for the top strand of the unmethylated DNA sequence, the most 3'-end of the primer hybridizes at the thymine residue which is derived from the cytosine of the CpG site to be analyzed, and the 5'-end of said second MSPE primer comprises a second unique sequence that does not hybridize to any DNA sequences in the sample;
(e) hybridizing the primer extension products from (d) to at least one pair of oligonucleotides, wherein the first oligonucleotide in the pair is complementary to said first unique sequence, and the second oligonucleotide in the pair is complementary to said second unique sequence; and
(f) determining the 5'-methylation status at the cytosine residue of the CpG site by comparing the hybridization intensity of the methylated DNA to the hybridization intensity of the unmethylated DNA in the sample.

In another aspect, the present invention provides a method for determining DNA methylation state or status at a specific CpG site or sites of interest, comprising the steps of:
(a) obtaining and/or using DNA from a sample to be analyzed;
(b) contacting the DNA with an agent that modifies unmethylated cytosine to uracil while leaving any 5'-methylated cytosine unchanged;
(c) amplifying the DNA using strand-specific primers;
(d) performing a primer extension reaction using at least one pair of MSPE primers that hybridize to the bottom strand of the amplified DNA, labeled dNTPs, and a DNA polymerase, wherein the 3'-end of the first MSPE primer in the pair comprises a polynucleotide sequence that is specific for the bottom strand of the methylated DNA, the most 3'-end of the primer hybridizes at the guanine residue complementary to the cytosine of the CpG site on the top strand, and the 5'-end of said first MSPE primer comprises a first unique sequence that does not hybridize to any DNA sequences in the sample; wherein the 3'-end of the second MSPE primer in the pair comprises a polynucleotide sequence that is specific for the bottom strand of the unmethylated DNA sequence, the most 3'-end of the primer hybridizes at the adenine residue which is derived from the cytosine of the CpG site on the top strand, and the 5'-end of said second MSPE primer comprises a second unique sequence that does not hybridize to any DNA sequences in the sample;
(e) hybridizing the primer extension products from (d) to at least one pair of oligonucleotides, wherein the first oligonucleotide in the pair is complementary to said first unique sequence, and the second oligonucleotide in the pair is complementary to said second unique sequence.
(f) determining the 5'-methylation status at the cytosine residue of the CpG site by comparing the hybridization intensity of the methylated DNA to the hybridization intensity of the unmethylated DNA in the sample.

In another aspect, the present invention provides a method for determining DNA methylation state or status at a specific CpG site or sites of interest, comprising the steps of:
(a) obtaining and/or using DNA from a sample to be analyzed;
(b) contacting the DNA with an agent that modifies unmethylated cytosine to uracil while leaving any 5'-methylated cytosine unchanged;
(c) amplifying the DNA using strand-specific primers;
(d) performing a primer extension reaction using at least one pair of methylation specific primer extension (MSPE) primers that hybridize to the top strand of the amplified DNA, dNTPs, labeled ddCTP and a DNA polymerase, wherein the 3'-end of the first MSPE primer in the pair comprises a polynucleotide sequence that is specific for the top strand of the methylated DNA, the most 3'-end of the primer hybridizes at the cytosine residue of the CpG site to be analyzed, and the 5'-end of said first MSPE primer comprises a first unique sequence that does not hybridize to any DNA sequences in the sample; wherein the 3'-end of the second MSPE primer in the pair comprises a polynucleotide sequence that is specific for the top strand of the unmethylated DNA sequence, the most 3'-end of the primer hybridizes at the thymine residue which is derived from the cytosine of the CpG site to be analyzed, and the 5'-end of said second MSPE primer comprises a second unique sequence that does not hybridize to any DNA sequences in the sample;
(e) hybridizing the primer extension products from (d) to at least one pair of oligonucleotides, wherein the first oligonucleotide in the pair is complementary to said first unique sequence, and the second oligonucleotide in the pair is complementary to said second unique sequence; and
(f) determining the 5'-methylation status at the cytosine residue of the CpG site by comparing the hybridization intensity of the methylated DNA to the hybridization intensity of the unmethylated DNA in the sample.

Alternatively, in another aspect, the present invention provides a method for determining DNA methylation state or status at a specific CpG site or sites of interest, comprising the steps of:
(a) obtaining and/or using DNA from a sample to be analyzed;
(b) contacting the DNA with an agent that modifies unmethylated cytosine to uracil while leaving any 5'-methylated cytosine unchanged;
(c) amplifying the DNA using strand-specific primers;
(d) performing a primer extension reaction using at least one pair of methylation specific primer extension (MSPE) primers that hybridize to the top strand of the amplified DNA, mixture of labeled dNTPs and ddNTPs, mixture of unlabeled dNTPs and ddNTPs, and a DNA polymerase, wherein the 3'-end of the first MSPE primer in the pair comprises a polynucleotide sequence that is specific for the top strand of the methylated DNA, the most 3'-end of the primer hybridizes at the cytosine residue of the CpG site to be analyzed, and the 5'-end of said first MSPE primer comprises a first unique sequence that does not hybridize to any DNA sequences in the sample; wherein the 3'-end of the second MSPE primer in the pair comprises a polynucleotide sequence that is specific for the top strand of the unmethylated DNA sequence, the most 3'-end of the primer hybridizes at the thymine residue which is derived from the cytosine of the CpG site to be analyzed, and the 5'-end of said second MSPE primer comprises a second unique sequence that does not hybridize to any DNA sequences in the sample;
(e) hybridizing the primer extension products from (d) to at least one pair of oligonucleotides, wherein the first oligonucleotide in the pair is complementary to said first unique sequence, and the second oligonucleotide in the pair is complementary to said second unique sequence; and
(f) determining the 5'-methylation status at the cytosine residue of the CpG site by comparing the hybridization intensity of the methylated DNA to the hybridization intensity of the unmethylated DNA in the sample.

In another aspect, the present invention provides a method for determining DNA methylation state or status at a specific CpG site or sites of interest, comprising the steps of
(a) obtaining and/or using DNA from a sample to be analyzed;
(b) contacting the DNA with an agent that modifies unmethylated cytosine to uracil while leaving any 5'-methylated cytosine unchanged;
(c) amplifying the DNA using strand-specific primers;
(d) performing a primer extension reaction using at least one pair of MSPE primers that hybridize to the bottom strand of the amplified DNA, mixture of labeled dNTPs and ddNTPs, mixture of unlabeled dNTPs and ddNTPs, and a DNA polymerase, wherein the 3'-end of the first MSPE primer in the pair comprises a polynucleotide sequence that is specific for the bottom strand of the methylated DNA, the most 3'-end of the primer hybridizes at the guanine residue complementary to the cytosine of the CpG site on the top strand, and the 5'-end of said first MSPE primer comprises a first unique sequence that does not hybridize to any DNA sequences in the sample; wherein the 3'-end of the second MSPE primer in the pair comprises a polynucleotide sequence that is specific for the bottom strand of the unmethylated DNA sequence, the most 3'-end of the primer hybridizes at the adenine residue which is derived from the cytosine of the CpG site on the top strand, and the 5'-end of said second MSPE primer comprises a second unique sequence that does not hybridize to any DNA sequences in the sample;
(e) hybridizing the primer extension products from (d) to at least one pair of oligonucleotides, wherein the first oligonucleotide in the pair is complementary to said first unique sequence, and the second oligonucleotide in the pair is complementary to said second unique sequence.
(f) determining the 5'-methylation status at the cytosine residue of the CpG site by comparing the hybridization intensity of the methylated DNA to the hybridization intensity of the unmethylated DNA in the sample.

Alternatively, in another aspect, the present invention provides a method for determining DNA methylation state or status at a specific CpG site or sites of interest, comprising the steps of
(a) obtaining and/or using a DNA from a sample to be analyzed;
(b) contacting the DNA with an agent that modifies unmethylated cytosine to uracil while leaving any 5'-methylated cytosine unchanged;
(c) amplifying the DNA using strand-specific primers;
(d) performing a primer extension reaction using at least a pair of MSPE primers that hybridize to the top strand of the amplified DNA, at least one labeled reverse primer, dNTPs, and a DNA polymerase, wherein the 5'-end of the first MSPE primer in the pair comprises a first unique sequence that does not hybridize to any DNA sequences in the sample, and the 3'-end of the first MSPE primer comprises a polynucleotide sequence that is specific for the top strand of the methylated DNA, the most 3'-end of the primer hybridizes at the cytosine residue of the CpG site to be analyzed; wherein the 5'-end of the second MSPE primer in the pair comprises a second unique sequence that does not hybridize to any DNA sequences in the sample, and the 3'-end of the second MSPE primer in the pair comprises a polynucleotide sequence that is specific for the top strand of the unmethylated DNA, the most 3'-end of the primer hybridizes at the thymine residue which is derived from the cytosine of the CpG site to be analyzed;
(e) hybridizing the primer extension products from (d) to at least a pair of oligonucleotides, wherein the first oligonucleotide in the pair is the same as the first unique sequence and the second oligonucleotide in the pair is the same as the second unique sequence; and
(f) determining the 5'-methylation status at the cytosine residue of the CpG site by comparing the hybridization intensity of the methylated DNA to the hybridization intensity of the unmethylated DNA in the sample.

In another aspect, the present invention provides a method for determining DNA methylation state or status at a specific CpG site or sites of interest, comprising the steps of:
(a) obtaining and/or using a DNA from a sample to be analyzed;
(b) contacting the DNA with an agent that modifies unmethylated cytosine to uracil while leaving any 5'-methylated cytosine unchanged;
(c) amplifying the DNA using strand-specific primers;
(d) performing a primer extension reaction using at least a pair of MSPE primers that hybridize to the bottom strand of the amplified DNA, at least one labeled reverse primer, dNTPs, and a DNA polymerase, wherein the 5'-end of the first MSPE primer in the pair comprises a first unique sequence that does not hybridize to any DNA sequences in the sample, and the 3'-end of the first MSPE primer comprises a polynucleotide sequence that is specific for the bottom strand of the methylated DNA, the most 3'-end of the primer hybridizes at the guanine residue complementary to the cytosine of the CpG site on the top strand; wherein the 5'-end of the second MSPE primer in the pair comprises a second unique sequence that does not hybridize to any DNA sequences in the sample, and the 3'-end of the second MSPE primer in the pair comprises a polynucleotide sequence that is specific for the bottom strand of the unmethylated DNA, the most 3'-end of the primer hybridizes at the adenine residue which is derived from the cytosine of the CpG site on the top strand;
(e) hybridizing the primer extension products from (d) to at least a pair of oligonucleotides, wherein the first oligonucleotide in the pair is the same as the first unique sequence and the second oligonucleotide in the pair is the same as the second unique sequence; and
(f) determining the 5'-methylation status at the cytosine residue of the CpG site by comparing the hybridization intensity of the methylated DNA to the hybridization intensity of the unmethylated DNA in the sample.

Any nucleic acid sample, in purified or non-purified form, can be utilized in accordance with the present invention, provided that it contains, or is suspected of containing a nucleic acid sequence containing the CpG-containing nucleic acids. In many genes, the CpG islands begin just upstream of a promoter and extend downstream into the transcribed region. Methylation of a CpG island at a promoter usually prevents expression of the gene. The islands can also surround the 5' region of the coding region of the gene as well as the 3' region of the coding region. Thus, CpG islands can be found in multiple regions of a nucleic acid sequence including upstream of coding sequences in a regulatory region including a promoter region, in the coding regions (e.g., exons), downstream of coding regions in, for example, enhancer regions, and in introns. In general, the CpG-containing nucleic acid is DNA. However, invention methods may employ, for example, samples that contain DNA, or DNA and RNA, including messenger RNA, wherein DNA or RNA may be single stranded or double stranded, or a DNA-RNA hybrid may be included in the sample. A mixture of nucleic acids may also be employed. The specific nucleic acid sequence to be detected may be a fraction of a larger molecule or can be present initially as a discrete molecule, so that the specific sequence constitutes the entire nucleic acid. It is not necessary that the sequence to be studied be present initially in a pure form; the nucleic acid may be a minor fraction of a complex mixture, such as contained in whole human DNA. The nucleic acids may be naturally occurring or non-naturally occurring. Additionally, their methylation status may be the result of *in vivo* processes, or of experimental manipulations (e.g., deliberate exposure to a putative DNA damaging agent, or a putative methylating agent). In a preferred embodiment, the nucleic acids in the sample is genomic DNA.

The methods of isolating nucleic acids are well known in the art and suitable methods can be found in standard molecular biology textbooks.

The sample containing DNA for detection of methylated CpG may be obtained from any source. Preferably, the sample may be obtained from cell lines, blood, sputum, stool, urine, serum, cerebro-spinal fluid, hair, tissue embedded in paraffin, for example, tissue from eyes, intestine, kidneys, brain, heart, prostate, lungs, breast or liver, histological slides, and all possible combinations thereof.

According to the present invention, it is preferred that the agent used to convert the unmethylated cytosine into uracil is a bisulfite compound. Preferably, the agent used for modifying unmethylated cytosine is sodium bisulfite. However, other agents that similarly modify unmethylated cytosine, but not methylated cytosine, may also be used in the methods of the present invention. Bisulfite (NaHSO₃) reacts readily with the 5,6-double bond of cytosine, but poorly with methylated cytosine. Cytosine reacts with the bisulfite ion to form a sulfonated cytosine reaction intermediate, which is susceptible to deamination, giving rise to a sulfonated uracil. The sulfonate group can be removed under alkaline conditions, resulting in the formation of uracil. Uracil is recognized as a thymine by Taq polymerase and therefore upon PCR, the resultant amplified product contains cytosine only at the position where 5-methylcytosine occurs in the starting template DNA.

In step (c) of the methods of the present invention, various methods known in the art may be used to amplify the nucleic acids. Amplification methods used in the present invention include, but not limited to PCR and LCR etc. In addition, other amplification methods that can be used according to the present invention are isothermic amplification, primer extension reactions, rolling-circle amplification, and all other amplification reactions known to one skilled in the art.

PCR or polymerase chain reaction, as described in U.S. Pat. Nos. 4,683,195 and 4,683,202, is a method of increasing the concentration of a segment of target nucleic acid sequence in a mixture of nucleic acids without cloning or purification. This technology provides one approach to the problems of low target sequence concentration. PCR can be used to directly increase the concentration of the target to an easily detectable level. This process for amplifying the target sequence involves introducing a molar excess of two oligonucleotide primers that are complementary to their respective strands of the double-stranded target sequence to the DNA mixture containing the desired target sequence. The mixture is denatured and then allowed to hybridize. Following hybridization, the primers are extended with polymerase so as to form complementary strands. The steps of denaturation, hybridization, and polymerase extension can be repeated as often as needed, in order to obtain relatively high concentrations of a segment of the desired target sequence. The length of the segment of the desired target sequence is determined by the relative positions of the primers with respect to each other, and, therefore, this length is a controllable parameter. Because the desired segments of the target sequence become the dominant sequences (in terms of concentration) in the mixture, they are said to be "PCR-amplified."

The ligase chain reaction (LCR; sometimes referred to as "Ligase Amplification Reaction" (LAR) described by Barany, *Proc. Natl. Acad. Sci. USA*, 88:189 (1991); Barany, *PCR Methods and Applic*., 1:5 (1991); and Wu and Wallace, *Genomics* 4:560(1989) has developed into a well- recognized alternative method for amplifying nucleic acids. In LCR, four oligonucleotides, two adjacent oligonucleotides which uniquely hybridize to one strand of target DNA, and a complementary set of adjacent oligonucleotides, that hybridize to the opposite strand are mixed and DNA ligase is added to the mixture. Provided that there is complete complementarity at the junction, ligase will covalently link each set of hybridized molecules. Importantly, in LCR, two probes are ligated together only when they base-pair with sequences in the target sample, without gaps or mismatches. Repeated cycles of denaturation, hybridization and ligation amplify a short segment of DNA. LCR has also been used in combination with PCR to achieve enhanced detection of single- base changes. Segev, PCT Public. No. W09001069 A1 (1990). However, because the four oligonucleotides used in this assay can pair to form two short ligatable fragments, there is the potential for the generation of target-independent background signal. The use of LCR for mutant screening is limited to the examination of specific nucleic acid positions.

In one preferred embodiment, PCR is used to amplify the chemically treated nucleic acids. PCR amplification can be performed following standard protocols. For example, approximately 1-2 µl of the chemically treated DNA is used as a template for strand-specific PCR amplification in a region of interest. In a PCR reaction profile for amplifying a portion of a 5' CpG island, for example, a procedure of initial denaturation of 94-95°C for 3-10 minutes followed by a cycle of 94-95°C of 30 seconds, 60°C for 30 seconds, 72°C for 30 seconds for a total of 30-40 cycles. The PCR reactions are performed in 10-50 µl volumes under conditions of: about 50 ng bisulfite-converted DNA (less for micro dissected samples), 10 mM Tris-HCl (pH 8.3), 1.5-2.5 mM MgCl₂, 50 mM KCl, 200 µM of each of dNTP, 0.4 µM final concentration of each primer and 1 unit of Taq polymerase.

In one embodiment, the PCR amplification is conducted with at least a pair of PCR primers. The PCR primers used in the present invention should recognize both the methylated and unmethylated DNA templates. In other words, the PCR primers should be designed to anneal to areas not containing the methylated CpG sites if possible. However, the two PCR primers should span the CpG sites. According to the present invention, the PCR primers are preferably single-stranded for maximum efficiency in amplification. Preferably, the PCR primers are single-stranded DNA or RNA molecules that hybridize to a template nucleic acid sequence and prime enzymatic synthesis of the complementary nucleic acid strand. Furthermore, the PCR primers are complementary to a portion of a target molecule present in a pool of nucleic acid molecules.

The PCR primers of the present invention should have sufficient length. In one embodiment, the PCR primers may comprise a sequence that hybridizes under stringent conditions to at least about 7, preferably about 12, preferably about 15, more preferably about 25, 50, 75, 100, or more nucleotides, ideally about 17 to 40 nucleotides. As used herein, the term "hybridizes under stringent conditions" is intended to describe conditions for hybridization and washing under which nucleotide sequences at least 75%, 80%, 85%, preferably 90% identical to each other typically remain hybridized to each other. Such stringent conditions are known to those skilled in the art and can be found in sections 6.3.1-6.3.6 of *Current Protocols in Molecular Biology*, John Wiley & Sons, N.Y. (1989). In another embodiment, the PCR primer may comprise a sequence that hybridizes under moderately stringent conditions to at least about 7, preferably about 12, preferably about 15, more preferably about 25, 50, 75, 100, or more nucleotides. For purposes of illustration, suitable moderately stringent conditions for testing the hybridization of a polynucleotide of this invention with other polynucleotides include prewashing in a solution of 5 x SSC, 0.5% SDS, 1.0 mM EDTA (pH 8.0); hybridizing at 50°C to 60°C, 5 x SSC, overnight; followed by washing twice at 65°C for 20 minutes with each of 2 x, 0.5 x, and 0.2 x SSC containing 0.1% SDS. One skilled in the art will understand that the stringency of hybridization can be readily manipulated, such as by altering the salt content of the hybridization solution and/or the temperature at which the hybridization is performed.

A positive correlation exists between primer length and both the efficiency and accuracy with which a primer will anneal to a target sequence. In particular, longer sequences have a higher melting temperature (Tm) than do shorter ones, and are less likely to be repeated within a given target sequence, thereby minimizing promiscuous hybridization. Primer sequences with a high G-C content or that comprise palindromic sequences tend to self-hybridize, as do their intended target sites, since unimolecular, rather than bimolecular, hybridization kinetics are generally favored in solution. However, it is also important to design a primer that contains sufficient numbers of G-C nucleotide parings since each G-C pair is bound by three hydrogen bonds, rather than the two that are found in a A-T base pair.

PCR primers of the present invention are also designed to have a particular melting temperature (Tm) by the method of melting temperature estimation. Commercial programs, including Oligo^{™}, Primer Design and programs available on the internet, including Primer3 and Oligo Calculator can be used to calculate a Tm of a primer. Preferably, the Tm of a PCR primer is preferably between about 45°C and 70°C, and more preferably between about 55°C and 65°C. Preferably, the Tm of a primer is about 3-5°C higher than the Tm of the corresponding PCR primers.

It is contemplated that PCR primers of the present invention are prepared by synthetic methods, either chemical or enzymatic. Alternatively, such molecules or fragments thereof are naturally-occurring, and are isolated from their natural source or purchased from a commercial supplier.

In yet another embodiment, the present invention provides in one vessel, multiple pairs of primers, each anneals to a defined CpG region within one nucleic acid molecule. The multiple pairs of primers allow concurrent amplifications of different CpG sites in one vessel and produce multiple amplified nucleic acid fragments. The multiple amplified nucleic acid fragments can then be detected and quantified in a high throughput manner.

The amplified nucleic acids are preferably identified as either methylated or non-methylated by methylation specific primer extension (MSPE) reaction. The primer extension reaction is conducted using standard techniques known in the art, but with MSPE primers. For example, in one embodiment, the MSPE reactions are performed at 95°C for 10 minute, followed by 95°C for 30 second, 60°C for 30 second, and 72°C for 30 second, for 30-40 cycles. In the step where the MSPE reactions are carried out on the amplified nucleic acids, a set of MSPE primers are used either separately in different vessels or simultaneously in one vessel. Generally, MSPE primers used in the primer extension reaction are designed to hybridize to sequences that originally contain CpG dinucleotides to be analyzed. The selection and design of a MSPE primer for optimal extension reaction are well know to one of skill in the ordinary art. Preferably, the MSPE primers comprise sequences that hybridize under stringent conditions to at least about 7, preferably about 12, more preferably about 25, 50, 75, 100, 125, 150, 175, 200, or more consecutive nucleic acid sequences.

It is contemplated that the present invention should encompass more than one class of MSPE primers. For analysis of one defined CpG site, preferably at least two classes of MSPE primers are included in the extension reaction. For example, one class of MSPE primer is methylation specific and annealing to the methylated CpG site(s) and the other class of MSPE primer is unmethylation specific and annealing to the unmethylated CpG site(s) of the amplified nucleic acids. Each class of MSPE primer consists of two moieties. The 5' end portion anneals to a unique/adapter sequence that does not anneal to any of the amplified nucleic acids, and the 3' end portion anneals to the CpG site(s) to be analyzed on the amplified nucleic acids. In one embodiment, the most 3' end of the MSPE primer anneals at the CpG site(s) to be analyzed. The term "the most 3' end" as used herein, refers to the last few nucleotides at the 3' end of the MSPE primer, preferably the last four nucleotides, more preferably the last two nucleotides, and most preferably the last nucleotide, with the last nucleotide at of the MSPE primer annealing at the cytosine position of the CpG site(s) to be analyzed. For example, in a methylation specific MSPE primer, the last nucleotide anneals at the cytosine of a CpG site on the top strand or at the guanine on the bottom strand, whereas in a unmethylation specific MSPE primer, the last nucleotide anneals at the thymine of TpG on the top strand or at the adenine on the bottom strand because unmehtylated cytosine of the CpG site has been converted into uracil and amplified as thymine.

The unique/adapter (sometimes referred to in the art as "zip code") sequence is a sequence generally exogeneous to the target sequences, e.g., artificial, that is designed to be substantially complementary (and preferably perfectly complementary) to a capture probe on a detection device. Particularly, the capture probe is immobilized to a solid support that can include microspheres or planar substrates such as plastic or glass slides. The length of the adapter sequence will vary depending on the desired "strength" of binding and the number of different adapters desired. In a preferred embodiment, the adapter sequence ranges from about 6 to about 500 nucleotides in length, more preferably from about 8 to about 100 nucleotides, and most preferably from about 10 to about 25 nucleotides in length. Generally, each adapter sequence uniquely identifies an amplified nucleic acid fragment. Detection of the adapter sequence is accomplished following hybridization with a capture probe that is complementary to the adapter sequence.

In one embodiment, the primer extension reaction is conducted by using MSPE primers that anneal to the top strand of the amplified DNA and by using the labeled dNTPs, wherein dATP, dTTP, and dCTP are labeled. In another embodiment, the primer extension reaction is conducted by using MSPE primers that anneal to the bottom strand of the amplified DNA and by using the labeled dNTPs, wherein dATP, dTTP, and dGTP are labeled.

In another embodiment, the primer extension reaction is conducted by using MSPE primers that anneal to the top strand of the amplified DNA and by using labeled ddCTP. In another embodiment, the primer extension reaction is conducted by using MSPE primers that anneal to the top strand of the amplified DNA and by using mixture of labeled dNTPs and ddNTPs, and mixture of unlabeled dNTPs and ddNTPs. In another embodiment, the primer extension reaction is conducted by using MSPE primers that anneal to the bottom strand of the amplified DNA and by using mixed labeled and unlabeled dNTPs and ddNTPs.

Alternatively, in another embodiment, the primer extension reaction is conducted by using MSPE primers that anneal to the top strand of the amplified DNA, the labeled reverse primers, and the unlabeled dNTPs. The reverse primers as used herein, refers to a primer that is complementary to the extension strand by the MSPE primers. The labeled reverse primers will extend in the opposite direction of the MSPE primer using the MSPE extension strand as template and stop at the 5' end of the MSPE primers. In another embodiment, the primer extension reaction is conducted by using MSPE primers that anneal to the bottom strand of the amplified DNA, the labeled reverse primers, and the unlabeled dNTPs.

The dNTPs, ddNTPs or reverse primers that are incorporated into the extension products can be labeled with a detectable label. The detectable label can comprise a radiolabel, a fluorescent label, a luminescent label, an antibody linked to a nucleotide that can be subsequently detected, a hapten linked to a nucleotide that can be subsequently detected, or any other nucleotide or modified nucleotide that can be detected either directly or indirectly. In one embodiment, dNTPs, ddNTPs or reverse primers are labeled with radioisotopes. The radiolabeled extension product can be detected by the presence of radioactivity. For example, dNTPs, ddNTPs or reverse primers can be labeled with ³²P, and the extension product can be analyzed by denaturing polyacrylamide gel and phosphorimage analysis. In another embodiment, dNTPs, ddNTPs or reverse primers are labeled with fluorescent agents. In one preferred embodiment, dNTPs, ddNTPs or reverse primers are labeled with Cyanine 3, Cyanine 5, phycoerythrin, Alexa 532, fluorescein, TAMRA, tetramethyl thodamine, fluorescent nucleotides, digoxigenin, or the like. In one more preferred embodiment, dNTPs, ddNTPs or reverse primers are labeled with biotin. When the biotin is used for labeling, the extension product can be captured by capture probe-attached microspheres and quantified by fluorescent methods.

In a preferred embodiment, the detectable label is a primary label that can be directly detected, such as fluorophore. In another preferred embodiment, the detectable label is a secondary label that can be indirectly detected. Such label includes but not limited to, one of the binding pairs such as biotin/streptavidin, chemically modifiable moieties, nuclease inhibitors, enzymes such as horseradish peroxidase, alkaline phosphatases, lucifierases, etc. Suitable binding pairs include but not limited to, antigens and antibodies, proteins and small molecules, enzymes and substrates or inhibitors, receptor and ligands, carbohydrates and their binding partners, and nucleic acid and nucleic acid binding proteins, etc. In general, the smaller of the pair is attached to the NTPs or dNTPs for incorporating into nucleic acids. Preferably, binding pairs include biotin (or imino-biotin) and streptavidin, digeoxinin and antibodies, and Prolinx_reagents. More preferably, the binding pairs comprise biotin or imino-biotin and streptavidin.

The MSPE extension product is further analyzed for relative quantification of cytosine methylation in a sample. The quantification includes first hybridizing the MSPE extension product to capture probes that are complementary to the adapter sequences on the MSPE extension product. Various hybridization procedures are well known in the art and the present invention is not limited to any particular hybridization procedure. In general, hybridization is usually carried out in solutions of high ionic strength (6 x SSC or 6 x SSPE) at a temperature 20-25°C, below the Tm. Specific examples of stringent hybridization conditions include 5 x SSPE, 50% formamide at 42°C. or 5 x SSPE at 68°C. Stringent wash conditions are often determined empirically in preliminary experiments, but usually involve a combination of salt and temperature that is approximately 12-25°C, below the Tm. One example of highly stringent wash conditions is 1 x SSC at 60°C. An example of very highly stringency wash conditions is 0.1 x SSPE, 0.1% SDS at 42°C. Meinkoth and Wahl, *Anal. Biochem*., 138:267-284 (1984). An example of extremely stringent wash conditions is 0.1 x SSPE, 0.1% SDS at 50-65°C. In one preferred embodiment, high stringency washing is carried out under conditions of 1 x SSC and 60°C. Another preferred embodiment, hybridization is carried at 40°C, by using 0.5xhybridization buffer, which was made from HEPES, sodium salt, 13.0 g/L, HEPES, free acid, 12.0 g/L, Lithium Lauryl Sulfate, 10 g/L, LiCl, 21.2 g/L, Bovine Serum Albumin, 10.0 g/L, Casein, 1.5 g/L, BRIJ-35, 10.0 g/L, MgCl₂, 2.0 g/L, ZnCl₂, 0.00136g/L, Sodium Azide, 0.50 g/L, Proclin-300, 0.50g/L, pH 7.5. Then Washing Buffer (0.4×SSC, 1.0 g/L SDS, 0.50 g/L Sodium Azide, 0.50 g/L Proclin-300, pH 7.7) is applied to the reaction several times at room temperature. As is well recognized in the art, various combinations of factors can result in conditions of substantially equivalent stringency. Such equivalent conditions are within the scope of the present inventive discovery.

In one embodiment, the capture probes are hybridized to the labeled polynucleotides under stringent, more typically highly stringent, or very stringent , or extremely stringent conditions. The capture probes comprise polynucleotides of about 5 to about 100, preferably about 6 to about 75, or about 8 to about 65, or about 10 to about 50, or about 15 to about 40, or about 16 to about 35, or about 18 to about 30 nucleotides in length. The capture probes can be readily synthesized by well known techniques in the art for the synthesis of polynucleotides such as those described in U.S. Pat. No. 4,973,679. Alternatively, the capture probes can be ordered from numerous commercial sources.

In another embodiment, the capture probes can be linked to a solid support, such as but not limited to, a microbead, a chromatography bead, an affinity bead, a gene chip, a planar waveguide, a membrane, a microliter plate, a glass plate, a plastic plate, or the like. In one more preferred embodiment, the capture probes are linked to a microbead, preferably Luminex microbeads. In one embodiment, the capture probes are linked to a single the microbeads by the well known carbodiimide coupling procedure. Luminex microbeads are extensively described in U.S. Pat. No. 6,268,222, Microbeads or microspheres are small discrete particles. The composition of the beads varys depending on the class of capture probe and the method of synthesis. Suitable bead compositions include those used in peptide, nucleic acid and organic moiety synthesis, including but not limited to, plastics, ceramics, glass, polysteyrene, 5 methylstyrene, acrylic polymers, paramagnetic materials, thorial sol, carbon graphite, titanium dioxide, latex or cross-linked dextrans such as Sepharose, cellulose, nylon, cross-linked micelles and Teflon. The microbeads incorporate polymeric nanoparticles that are stained with one or more fluorescent dyes. All of the nanoparticles in a given population are dyed with the same concentration of a dye, and by incorporating a known quantity of these nanoparticles into the microbeads. By varying the quantity and ratio of different populations of nanoparticles, it is possible to establish and distinguish a large number of discrete populations of microbeads (microspheres) with unique emission spectra. The fluorescent dyes used are of the general class known as cyanine dyes, with emission wavelengths between 550 nm and 900 nm. These dyes may contain methine groups; the number of methine groups influences the spectral properties of the dye. The monomethine dyes that are pyridines typically have a blue to blue-green fluorescence emission, while quinolines have a green to yellow-green fluorescence emission. The trimethine dye analogs are substantially shifted toward red wavelengths, and the pentamethine dyes are shifted even further, often exhibiting infrared fluorescence emission. However, any dye compatible with the composition of the beads can be used.

When a number of different microbeads are used in practicing the methods described herein, it is preferable, but not required, that the dyes have the same or overlapping excitation spectra, but possess distinguishable emission spectra. Multiple classes or populations of particles can be produced from just two dyes. The ratio of nanoparticle populations with red/orange dyes is altered by an adequate increment in proportion so that the obtained ratio does not optically overlap with the former ratio. In this way a large number of differently fluorescing microbead classes are produced.

Various methods can be employed to detect and quantify the relative methylation level at one or more CpG sites on a nucleic acid molecule. Detection systems used in the present invention may include but not limited to, a solid state detector, photomultiplier tube, photographic film, or eye, any of which may be used in conjunction with additional instrumentation such as a spectrometer, luminometer microscope, plate reader, fluorescent scanner, flow cytometer, or any combination thereof, to complete the detection system.

In one embodiment, microbeads are identified using a flow cytometer, for example a fluorescence-activated cell sorter, wherein the different classes of beads in a mixture can be physically separated from each other based on the fluorochrome identity, size and/or shape of each class of bead, and the presence of the target polynucleotide qualitatively or quantitatively determined based on the presence of the detectable label for each sorted pool containing beads of a particular class. Any flow cytometer capable of detecting both the particles and the label contained in the polynucleotide hybridized to the capture probe can be used. Flow cytometers with multiple excitation lasers and detectors are preferred. In one embodiment, the Luminex 100 flow cytometer is used. As is well known in the art, the exact setting necessary for optimum detection will vary with factors such as the flow cytometer used, the polynucleotide label used, and the particles used. Optimization of settings and conditions for the use of a flow cytometer for practicing the methods disclosed herein can be accomplished by the skilled technician without undue experimentation. General guidance on the use of flow cytometers can be found in texts such as Shapiro, *Practical Flow Cytometry*, 3rd ed., Wiley-Liss, 1995 and Jaroszeski et al., *Flow Cytometry Protocols*, Humana Press, 1998. An example of the use of fluorescent microbeads and flow cytometery can be found in Smith et al., *Clin. Chem*., 44:2054-2056 (1998). The use of flow cytometry is especially useful in the situation where greater than one class of particles and a plurality of capture probes are used to simultaneously to determine the presence of multiple target polynucleotides (multiplex analysis).

In one embodiment, the detection and quantification of relative methylation levels at one or more CpG sites on nucleic acid molecules include the use of microbeads conjugated with dyes, such as fluorescent dyes. For example, for one defined CpG site on a nucleic acid molecule, the MSPE products contain the nucleic acids of both methylated and unmethylated origins. Therefore, in detecting the relative methylation level of one CpG site, a given fluorescent signature is conjugated to a microbead that is linked to a particular capture probe, wherein the capture probe is complementary to a particular adapter sequence, said adapter sequence corresponding to the methylated state of a particular CpG site on a nucleic acid molecule, whereas another given fluorescent signature is conjugated to another microbead that is linked to another particular capture probe, wherein said capture probe is complementary to another particular adapter sequence, said adapter sequence corresponding to the methylated state of the same CpG site on the same nucleic acid molecule. The detection of the relative methylation level includes first identifying the fluorescent signature on microbeads, then determining and quantifying the intensity of the fluorescent signals on the labeled MSPE extension product. The relative methylation level at said CpG site is calculated by comparing the fluorescent signals of the methylated MSPE extension product with the fluorescent signals of the unmethylated MSPE extension product. As described above, the MSPE extension product is labeled with either the primary label or the secondary label.

Alternatively, the relative methylation levels at multiple CpG sites on a nucleic acid molecule or even on different nucleic acid molecules can be simultaneously determined by using the conjugated microbeads and following similar detection steps. For example, distinguishable adapter sequences each are linked to either methylated or unmethylated state of a nucleic acid molecule containing one CpG site. Moreover, distinguishable adapter sequences each are designed to correspond to each defined CpG site. Finally, a distinguishable microbead is conjugated with a dye specific for each distinguishable adapter sequence. Likewise, the detection step includes first identifying a distinguishable fluorescent signature on a microbead, then determining and quantifying the intensity of the fluorescent signals on the labeled MSPE extension product. As described above, the MSPE extension product is labeled with either the primary label or the secondary label.

In another embodiment, the detection and quantification of relative methylation levels at multiple CpG sites on nucleic acid molecules can also be performed in a high throughput manner, e.g., on microarrays. Microarray based analysis of the relative methylation levels enables working with hundreds of thousands of CpG sites simultaneously rather than one or a few CpG sites at a time. A DNA microarray is composed of an ordered set of DNA molecules of known sequences usually arranged in rectangular configuration in a small space such as 1 cm² in a standard microscope slide format. For example, an array of 200 x 200 would contain 40,000 spots with each spot corresponding to a probe of known sequence. Such a microarray can be potentially used to simultaneously monitor the expression of 40,000 nucleic acids in a given cell type under various conditions. The probes usually take the form of cDNA, ESTs or oligonucleotides. Most preferred are ESTs and oligonucleotides in the range of 30-200 bases long as they provide an ideal substrate for hybridization. There are two approaches to building these microarrays, also known as chips, one involving covalent attachment of pre-synthesized probes, the other involving building or synthesizing probes directly on the chip. The sample or test material usually consists of nucleic acids that have been amplified by PCR. PCR serves the dual purposes of amplifying the starting material as well as allowing introduction of fluorescent tags. For a detailed discussion of microarray technology, see e.g., Graves, *Trends Biotechnol.* 17: 127-134 (1999).

Methylation can also be detected by means of high-density microarrays. High-density microarrays are built by depositing an extremely minute quantity of DNA solutions at precise location on an array using high precision machines, a number of which are available commercially. An alternative approach pioneered by Packard Instruments, enables deposition of DNA in much the same way that ink jet printer deposits spots on paper. High-density DNA microarrays are commercially available from a number of sources such as Affymetrix, Incyte, Mergen, Genemed Molecular Biochemicals, Sequenom, Genomic Solutions, Clontech, Research Genetics, Operon and Stratagene. Currently, labeling for DNA microarray analysis involves fluorescence, which allows multiple independent signals to be read at the same time. This allows simultaneous hybridization of the same chip with two samples labeled with different fluorescent dyes. The calculation of the ratio of fluorescence at each spot allows determination of the relative change in the expression of each gene, or the relative methylation level herein, under two different conditions. For example, comparison between a normal tissue and a corresponding tumor tissue using the approach helps in identifying genes whose expression is significantly altered. Thus, the method offers a particularly powerful tool when the gene expression profile of the same cell is to be compared under two or more conditions. High-resolution scanners with capability to monitor fluorescence at various wavelengths are commercially available.

Since large numbers of classes of adapter sequences can be used simultaneously, each specific for a single target CpG site, it is possible to detect qualitatively or quantitatively, the relative methylation levels of hundreds of or thousands of CpG sites in one experiment. For example, different capture probes complementary to different adapter sequences are immobilized on a solid support, forming arrays, with different adapter sequences being located at different positions on the arrays. For detection purposes, mixtures of MSPE extension products deriving from different CpG sites are applied to the arrays, with each particular CpG site binding to a particular capture probe at particular location on the arrays. The signal intensity of the labeled MSPE product at a particular location can be determined with methods well known in the art, and the relative methylation levels at those CpG sites can be calculated by comparing the signal intensity at two locations corresponding to the methylation and unmethylation states of one particular CpG site. As described above, the MSPE extension product is labeled with either the primary label or the secondary label.

Another aspect of the present invention also provides a kit for the detection of relative methylation levels at one or more CpG sites on a nucleic acid molecule. The kit may include (1) an agent that modifies unmethylated cytosine nucleotides, (2) primers for amplification of the nucleic acid molecule, (3) MSPE primers as defined in claim 1 for the methylated CpG-containing nucleic acid (either labeled or unlabeled), (4) MSPE primers as defined in claim 1 for the unmethylated CpG-containing nucleic acid (either labeled or unlabeled), (5) labeled and unlabeled dNTPs, (6) labeled and unlabeled ddNTPs, and (7) labeled or unlabeled reverse primer. The kit may further include nucleic acid amplification buffer and reagents for MSPE reactions. Preferably, the agent that modifies unmethylated cytosine is a bisulfite compound.

Another aspect of the present invention also provides a method of determining a predisposition to a disease or disorder, or to diagnose and/or prognose of a disease or disorder, or to monitor therapeutic response of a drug or compound, in a subject comprising determining the relative methylation state at one or more CpG sites on a nucleic acid molecule, and comparing the relative methylation level in the subject to the relative methylation level of said nucleic acid molecule from a control subject (or standard) not having a predisposition to the disease or disorder, wherein a significant difference in the relative methylation level is indicative of the predisposition to the disease or disorder in the subject. According to a preferred aspect of the invention embodiments are excluded which refer to diagnostic methods practised on the human or animal body.

### Examples

### Example 1

The promoter sequence of p16 gene was used as model system. The sequence is listed below:

Genomic DNA from cell lines, HT29 and LNCaP, was used as methylation positive and negative controls, as they were confirmed experimentally previously.
Genomic DNA was modified initially according to Frommer method (Frommer, M., L. E. McDonald, D. S. Millar, C. M. Collis, F. Watt, G. W. Grigg, P. L. Molloy, and C. L. Paul, "A genomic sequencing protocol that yields a positive display of 5-methylcytosine residues in individual DNA strands," *Proc. Natl. Acad. Sci U.S.A.* 89:1827-1831 (1992)). In brief, 1 µg genomic DNA obtained from each cell line HT29 and LNCaP was diluted into 50 µl with distilled H₂O, add 5.5 µl of 2M NaOH, and incubated at 37°C for 10 minutes (to create single stranded DNA). Thirty µl of freshly prepared 10 mM hydroquinone (Sigma) was added to each tube. Five hundred twenty µl of freshly prepared 3M Sodium bisulfite (Sigma S-8890), pH 5.0 was then added. Reagents were thoroughly mixed and then covered with mineral oil and incubated at 50°C for 16 hours (at longer duration methylated C starts to convert to T). After removing the oil, 1 ml of Wizard DNA Cleanup Resin (Promega A7280) was added to each tube prior to applying the mixture to miniprep column in the DAN Wizard Cleanup kit. The column was washed with 2 ml of 80% isopropanol, and eluted with 50 µl of heated water (60-70°C). 5.5 µl of 3 M NaOH to was added to each tube, and incubated at room temperature for 5 minutes. Then 1 µl glycogen was added as carrier, 33 µl of 10 M NH₄Ac, and 3 volumes of ethanol for DNA precipitation. The pellet was spun down and washed with 70% ethanol, dried and resuspended in 20 µl water. One µl of DNA was used in each PCR reaction.

After bisulfite modification, the sequence changed to (Y denotes for C/T, as unmethylated cytosine been converted to uracil, and recognized as thymine; methylated cytosine remains intact as cytosine):

PCR reaction was conducted using standard protocol. One µl of the chemically treated DNA was used as a template PCR amplification. In a PCR reaction, a procedure of initial denaturation of 95°C for 10 minutes followed by a cycle of 95°C of 30 seconds, 60°C for 30 seconds, 72°C for 30 seconds for a total of 40 cycles was used. The PCR reactions were performed in 50 µl volume, containing about 20 ng of bisulfite-converted DNA, 15 mM Tris-HCl (pH 8.0), 2.0 mM MgCl₂, 50 mM KCl, 200 µM of each of dNTP, 0.4 µM final concentration of each primer and 1 unit of AnipliTaq Gold DNA Polymerase. The primers were: p16Hmod1F 5' GAAGAAAGAGGAGGGGTTGG 3' (SEQ ID NO. 5)/p16Hmod1R 5' CTACAAACCCTCTACCCACC 3' (SEQ ID NO. 6). The amplicons were quantified by Agilent BioAnalyzer®.

A series mixture (Table 1.) of the two amplicons with varying ratio was used as template in a two-plex MSPE reaction using incorporation of biotinylated dGTP. The reaction started with an initial denaturation of 95°C for 10 minutes followed by a cycle of 95°C of 30 seconds, 60°C for 30 seconds, 72°C for 30 seconds for a total of 30 cycles. The 25 µl reaction contained: 2.5 µl of premixed amplicons of varying ratios (listed in Table 1), 10 mM Tris-HCl (pH 8.3), 1.5 mM MgCl₂, 50 mM KCl, 8 µM of each unlabeled dNTP, 4 µM Biotinylated dGTP, 100 nM final concentration of each MSPE primers, and 25 nM of each reverse primer, and 0.5 unit of AmpliTaq Gold polymerase. The primers were:
15183 CE-Methyl_136U22 5' GAF GCT JTJ ACC TFA JAG CJT TCG TTT CGG TTG ATT GGT TGG TTA C 3' (SEQ ID NO. 7), wherein the first 24 nucleotides are zip code sequence.
15184 Methyl_252L23 5' GCT ACA AAC CCT CTA CCC ACC TA 3' (SEQ ID NO. 8)
15185 CE-unMethyl_134U24 5' JCG JCA TFA GCF TCT JGA GFA CGT TTT TGG TTG ATT GGT TGG TTA T 3' (SEQ ID NO. 9), wherein the first 22 nucleotides are zip code sequence.
15186 unMethyl_252L24 5' CAC TAC AAA CCC TCT ACC CAC CTA 3' (SEQ ID NO. 10)
(J = iso G, F = iso C, two AEGIS^{™} bases (isoC and isoG), unique non-standard base pairs, developed by EraGen Biosciences, licensed by the Bayer Corporation. Both "J" and "F" are designated as "n" on the sequence listing in compliance with WIPO standard. The uses of the symbols "J" and "F" also apply to SEQ ID NO's 10 and 11.)

**Table 1. DNA Template Amount in MSPE Assay**

| **Sample ID** | **1** | **2** | **3** | **4** | **5** | **6** | **7** | **8** | **9** | **10** | 1**1** |
|---|---|---|---|---|---|---|---|---|---|---|---|
| **Methylated Target (ng)** | 5.60 | 5.04 | 4.48 | 3.92 | 3.36 | 2.80 | 2.24 | 1.68 | 1.12 | 0.56 | 0.00 |
| **Unmethylated Target (ng)** | 0.00 | 0.84 | 1.68 | 2.52 | 3.36 | 4.20 | 5.04 | 5.88 | 6.72 | 7.56 | 8.40 |
| **Expected Methylation (%)** | 100.00 | 85.71 | 72.73 | 60.87 | 50.00 | 40.00 | 30.77 | 22.22 | 14.29 | 6.90 | 0.00 |

After the reaction, MSPE products were transferred into a bead pool containing Luminex® beads, which had been conjugated with amine derivatized oligos: 15189 CP32-3a 5' CGA AFG CTF TJA GGT FAF AGC JTC Sp-LCA 3' (SEQ ID NO. 11)/15190 CP33-3a 5' GTJ CTC FAG AJG CTJ ATG FCG F-Sp-LCA 3' (SEQ ID NO. 12), respectively. The conjugation was done using standard protocol, and the beads were resuspended at 5,000 µl in 1×TE buffer (10 mM Tris, 1 mM EDTA, pH 8.5). Quadruplicate hybridization was conducted at the same time. Basically, 4 µl of the MSPE product was transferred into a 50 µl hybridization reaction that contains 0.5xhybridization buffer and Luminex® beads 8 and 9 (at 2,000 each bead). The buffer was made from HEPES, sodium salt, 13.0 g/L, HEPES, free acid, 12.0 g/L, Lithium Lauryl Sulfate, 10 g/L, LiCl, 21.2 g/L, Bovine Serum Albumin, 10.0 g/L, Casein, 1.5 g/L, BRIJ-35, 10.0 g/L, MgCl₂, 2.0 g/L, ZnCl₂, 0.00136g/L, Sodium Azide, 0.50 g/L, Proclin-300, 0.50g/L, pH 7.5. The reaction was incubated at 96°C for 2 minute, 40°C for 30 minute. The beads were washed once each with 100 and 200 µl Washing Buffer (0.4xSSC, 1.0 g/L SDS, 0.50 g/L Sodium Azide, 0.50 g/L Proclin-300, pH 7.7). Fifty µl of 1:500 diluted Streptavidin-Phycoerythrin (SA-PE, 1mg/ml) was added into each reaction, mixed gently at room temperature for 15 minute (avoiding light) and washed again with 100 µl of Washing Buffer. The beads were resuspended in 80 µl TTL Buffer (50 mM Tris, 0.1 % Tween-20, 400 mM LiCl, pH 8.0), and measurement was taken on Luminex® 100^{™} instrument.

**Table 2. Readout (MFU) from Luminex® 100^{™}**

| | **Raw Readout (Avrg - Bkgd)** | | **Normalized Readout (Avrg - NTC)** | | **StDev (MFU)** | |
|---|---|---|---|---|---|---|
| **Sample ID** | **M** | **M** | **U** | **U** | **M** | **U** |
| **1** | 1568.45 | 18.03 | 1567.42 | 0.00 | 48.41 | 0.00 |
| **2** | 1283 | 401.48 | 1281.97 | 383.45 | 34.54 | 14.08 |
| **3** | 956.16 | 730.25 | 955.13 | 712.22 | 43.65 | 31.98 |
| **4** | 718.98 | 931.7 | 717.95 | 913.67 | 29.54 | 28.21 |
| **5** | 538.23 | 1153.28 | 537.2 | 1135.25 | 30.57 | 108.13 |
| **6** | 414.3 | 1271.95 | 413.27 | 1253.92 | 16.85 | 22.75 |
| **7** | 287.25 | 1392.17 | 286.22 | 1374.14 | 9.30 | 41.80 |
| **8** | 180.86 | 1585.12 | 179.83 | 1567.09 | 10.17 | 84.13 |
| **9** | 100.73 | 1563.43 | 99.7 | 1545.4 | 2.68 | 17.96 |
| **10** | 46.05 | 1601.39 | 45.02 | 1583.36 | 1.11 | 26.46 |
| **11** | 1.03 | 1573.37 | 0.00 | 1555.34 | 0.00 | 74.75 |

| | | | | | | |
|---|---|---|---|---|---|---|
| MFU, Mean Fluorescence Unit; M, Methylated; U, Unmethylated; NTC, Non_Template Control | | | | | | |

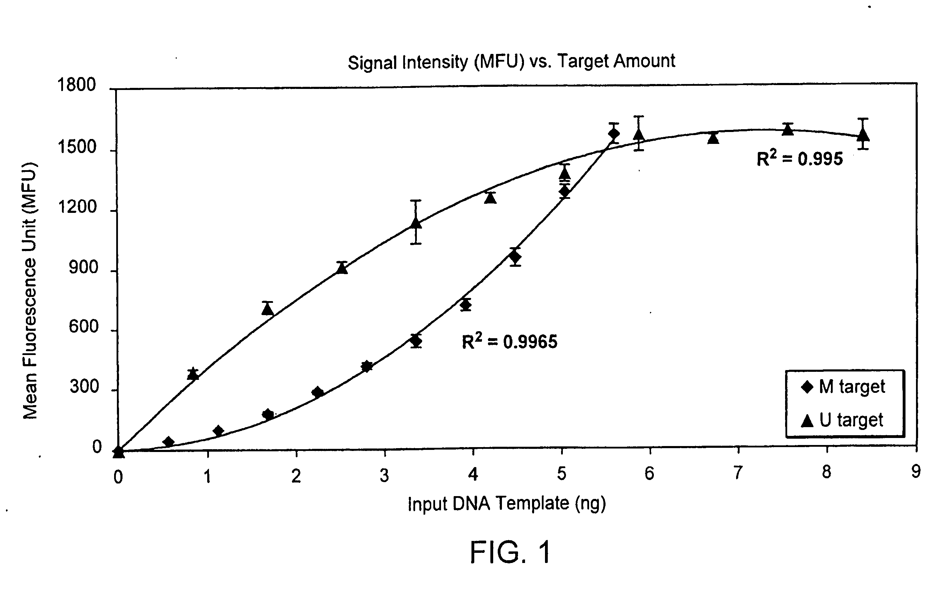

The results showed excellent readout for both methylated and unmethylated signals [Fig.1] with limited amount of template (ng level, see Table 2). The background of methylated and unmethylated signal was minimal, 1.03 and 18.03 MFU, respectively. The signal-to-noise ratio was >22 fold (401.48/18.03, see Table 2). Both methylated and unmethylated signals were clearly discriminated, with minimal variability (< 9.53%) between quadruplet reactions. The signal intensity of both are in comparable range, and fit a binomal curve tightly (R² > 0.995) [Fig. 1].

### Example 2

The promoter sequence of p16 gene was used as model system. The sequence is listed below:

Genomic DNA from cell lines, HT29 and LNCaP, was used as methylation positive and negative controls, as they were confirmed experimentally previously.

Genomic DNA was modified initially according to Frommer method (Frommer, M., L. E. McDonald, D. S. Millar, C. M. Collis, F. Watt, G. W. Grigg, P. L. Molloy, and C. L. Paul, "A genomic sequencing protocol that yields a positive display of 5-methylcytosine residues in individual DNA strands," *Proc. Natl. Acad. Sci U.S.A.* 89:1827-1831 (1992)). In brief, 1 µg Genomic DNA obtained from each cell line HT29 and LNCaP was diluted into 50 µl with distilled H₂O, add 5.5 µl of 2M NaOH, and incubated at 37°C for 10 minutes (to create single stranded DNA). Thirty µl of freshly prepared 10 mM hydroquinone (Sigma) was added to each tube. Five hundred twenty µl of freshly prepared 3M Sodium bisulfite, pH 5.0, (Sigma S-8890) was then added. Reagents wee thoroughly mixed and then covered with mineral oil and incubated at 50°C for 16 hours (at longer duration methylated C starts to convert to T). After removing the oil, 1 ml of Wizard DNA Cleanup Resin (Promega A7280) was added to each tube prior to applying the mixture to miniprep column in the DNA Wizard Cleanup kit. The column was washed with 2 ml of 80% isopropanol, and eluted with 50 µl of heated water (60-70°C). 5.5 µl of 3 M NaOH was added to each tube, and incubated at room temperature for 5 minutes. Then 1 µl glycogen was added as carrier, 33 µl of 10 M NH₄Ac, and 3 volumes of ethanol for DNA precipitation. The pellet was spun down and washed with 70% ethanol, dried and resuspended in 20 µl water. One µl of DNA was used in each PCR reaction.

After bisulfite modification, the sequence changed to (Y denotes for C/T, as unmethylated cytosine been converted to uracil, and recognized as thymine; methylated cytosine remains intact as cytosine):

PCR reaction was conducted using standard protocol. One µl of the chemically treated DNA was used as a template PCR amplification. In a PCR reaction, a procedure of initial denaturation of 95°C for 10 minutes followed by a cycle of 95°C of 30 seconds, 60°C for 30 seconds, 72°C for 30 seconds for a total of 40 cycles was used. The PCR reactions were performed in 50 µl volume, containing about 20 ng of bisulfite-converted DNA, 15 mM Tris-HCl (pH 8.0), 2.0 mM MgCl₂, 50 mM KCl, 200 µM of each of dNTP, 0.4 µM final concentration of each primer and 1 unit of AmpliTaq Gold DNA Polymerase. The primers were: p16Hmod1F 5' GAAGAAAGAGGAGGGGTTGG 3' (SEQ ID NO. 5) /p16Hmod1R 5' CTACAAACCCTCTACCCACC 3' (SEQ ID NO. 6). The amplicons were quantified by Agilent BioAnalyzer®.

A series mixture (Table 1.) of the two amplicons with varying ratio was used as template in a two-plex MSPE reaction using incorporation of biotinylated dGTP. The reaction started with an initial denaturation of 95°C for 10 minutes followed by a cycle of 95°C of 30 seconds, 60°C for 30 seconds, 72°C for 30 seconds for a total of 30 cycles. The 25 µl reaction contained: 2.5 µl of premixed amplicons of varying ratios (listed in Table 1), 10 mM Tris-HCl (pH 8.3),1.5 mM MgCl₂, 50 mM KCl, 8 µM of each unlabeled dNTP, 4 µM Biotinylated dGTP, 100 nM final concentration of each MSPE primers, and 25 nM of each reverse primer, and 0.5 unit of AmpliTaq Gold polymerase. The primers were:
15183 CE-Methyl_136U22 5' GAF GCT JTJ ACC TFA JAG CJT TCG TTT CGG TTG ATT GGT TGG TTA C 3' (SEQ ID NO. 7), wherein the first 24 nucleotides are zip code sequence.
15184 Methyl_252L23 5' GCT ACA AAC CCT CTA CCC ACC TA 3' (SEQ ID NO. 8)
15185 CE-unMethyl_134U24 5' JCG JCA TFA GCF TCT JGA GFA CGT TTT TGG TTG ATT GGT TGG TTA T 3' (SEQ ID NO. 9), wherein the first 22 nucleotides are zip code sequence.
15186 unMethyl_252L24 5' CAC TAC AAA CCC TCT ACC CAC CTA 3' (SEQ ID NO. 10)

After the reaction, MSPE product were transferred into a bead pool containing Luminex® beads, which had been conjugated with amine derivatized oligos: 15189 CP32-3a 5' CGA AFG CTF TJA GGT FAF AGC JTC Sp-LCA 3' (SEQ ID NO. 11)/15190 CP33-3a 5' GTJ CTC FAG AJG CTJ ATG FCG F-Sp-LCA 3' (SEQ ID NO. 12), respectively. The conjugation was done using standard protocol, and the beads were resuspended at 5,000 µl in 1×TE buffer (10 mM Tris, 1 mM EDTA, pH 8.5). Quadruplicate hybridization was conducted at the same time. Basically, 4 µl of the MSPE product was transferred into a 50 µl hybridization reaction that contains 0.5×hybridization buffer and Luminex® beads 8 and 9 (at 2,000 each bead). The buffer was made from HEPES, sodium salt, 13.0 g/L, HEPES, free acid, 12.0 g/L, Lithium Lauryl Sulfate, 10 g/L, LiCl, 21.2 g/L, Bovine Serum Albumin, 10.0 g/L, Casein, 1.5 g/L, BRIJ-35, 10.0 g/L, MgCl₂, 2.0 g/L, ZnCl₂, 0.00136g/L, Sodium Azide, 0.50 g/L, Proclin-300, 0.50g/L, pH 7.5. The reaction was incubated at 96°C for 2 minute, 40°C for 30 minute. The beads were washed once each with 100 and 200 µl Washing Buffer (0.4xSSC, 1.0 g/L SDS, 0.50 g/L Sodium Azide, 0.50 g/L Proclin-300, pH 7.7). Fifty µl of 1:500 diluted Streptavidin-Phycoerythrin (SA-PE, 1mg/ml) was added into each reaction, mixed gently at room temperature for 15 minute (avoiding light) and washed again with 100 µl of Washing Buffer. The beads were resuspenede in 80 µl TTL Buffer (50 mM Tris, 0.1 % Tween-20, 400 mM LiCl, pH 8.0), and measurement was taken on Luminex® 100™ instrument.

**Table 3. Expected vs. Observed Percentage of Methylation**

| **Sample ID** | **1** | **2** | **3** | **4** | **5** | **6** | **7** | **8** | **9** | **10** | **11** |
|---|---|---|---|---|---|---|---|---|---|---|---|
| **Expected Ratio% M/(M+U)** | 100.00 | 85.71 | 72.73 | 60.87 | 50.00 | 40.00 | 30.77 | 22.22 | 14.29 | 6.90 | 0.00 |
| **Observed Ratio% M/(M+U)** | 98.86 | 76.17 | 56.70 | 43.56 | 31.82 | 24.57 | 17.10 | 10.24 | 6.05 | 2.80 | 0.07 |

| | | | | | | | | | | | |
|---|---|---|---|---|---|---|---|---|---|---|---|
| M, Methylated; U, Unmethylated | | | | | | | | | | | |

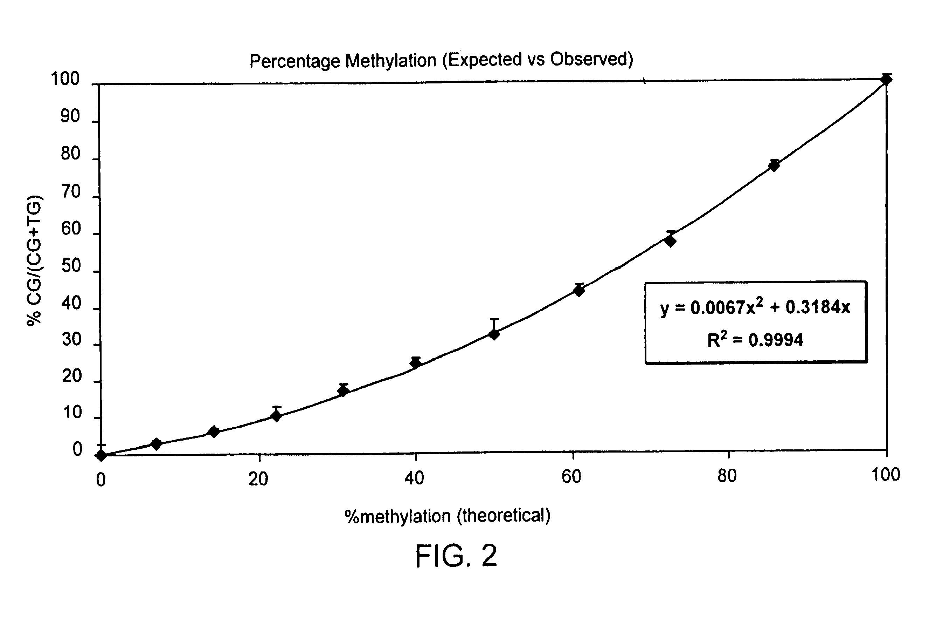

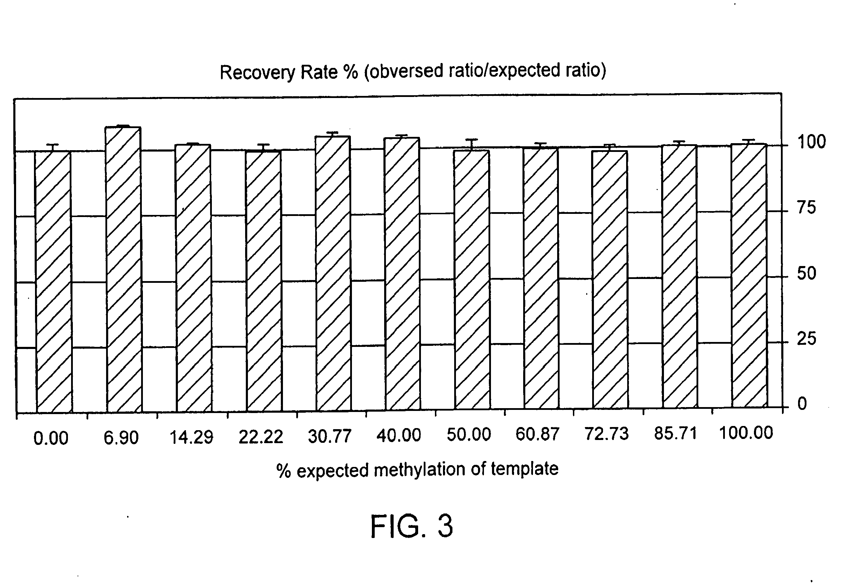

The expected ratio vs. input template ratio fits a binomial curve tightly (R² = 0.9994), demonstrating the high quantitative capability of the assay [Fig. 2]. The ratio was calculated directly from the Luminex® 100^{™} readout using the standard curve. The predictive power is nearly 100%, independent of the composition of the target (from 0 - 100% methylation) [Fig. 3].

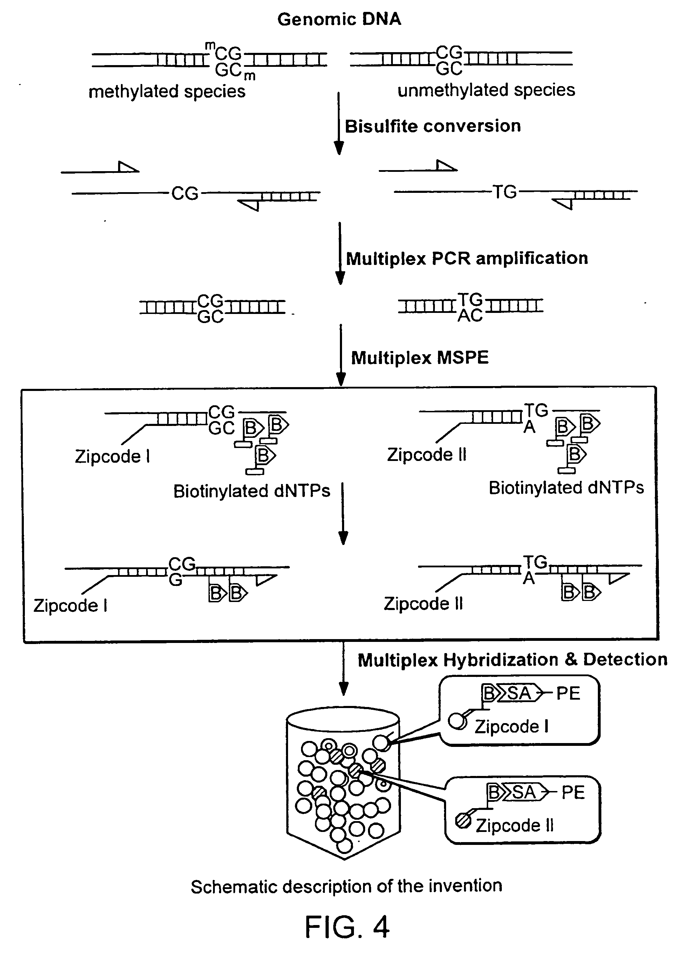

A schematic description of the invention is shown in Fig. 4.

### Other embodiments

Other embodiments will be evident to those of skill in the art. It should be understood that the foregoing detailed description is provided for clarity only and is merely exemplary.

## Claims

1. A method of detecting DNA 5'-methylation at a cytosine residue of a CpG site in a nucleic acid sequence, comprising:
(a) obtaining DNA from a sample to be analyzed;
(b) contacting the DNA with a bisulfite compound;
(c) amplifying the DNA using strand-specific primers;
(d) performing a primer extension reaction using at least one pair of methylation specific primer extension (MSPE) primers that hybridize to the top strand of the amplified DNA, labeled dNTPs, and a DNA polymerase, wherein the 3'-end of the first MSPE primer in the pair comprises a polynucleotide sequence that is specific for the top strand of the methylated DNA, the 3' terminal residue of the primer hybridizes at the cytosine residue of the CpG site to be analyzed, and the 5'-end of said first MSPE primer comprises a first unique sequence that does not hybridize to any DNA sequences in the sample; wherein the 3'-end of the second MSPE primer in the pair comprises a polynucleotide sequence that is specific for the top strand of the unmethylated DNA sequence, the 3' terminal residue of the primer hybridizes at the thymine residue which is derived from the cytosine of the CpG site to be analyzed, and the 5'-end of said second MSPE primer comprises a second unique sequence that does not hybridize to any DNA sequences in the sample;
(e) hybridizing the primer extension products from (d) to at least one pair of oligonucleotides, wherein the first oligonucleotide in the pair is complementary to said first unique sequence, and the second oligonucleotide in the pair is complementary to said second unique sequence; and
(f) determining the 5'-methylation status at the cytosine residue of the CpG site by comparing the hybridization intensity of the methylated DNA to the hybridization intensity of the unmethylated DNA in the sample;
or wherein
(d) the primer extension reaction is performed using at least one pair of MSPE primers that hybridize to the bottom strand of the amplified DNA, labeled dNTPs, and a DNA polymerase, wherein the 3'-end of the first MSPE primer in the pair comprises a polynucleotide sequence that is specific for the bottom strand of the methylated DNA, the 3' terminal residue of the primer hybridizes at the guanine residue complementary to the cytosine of the CpG site on the top strand, and the 5'-end of said first MSPE primer comprises a first unique sequence that does not hybridize to any DNA sequences in the sample; wherein the 3'-end of the second MSPE primer in the pair comprises a polynucleotide sequence that is specific for the bottom strand of the unmethylated DNA sequence, the 3' terminal residue of the primer hybridizes at the adenine residue which is derived from the cytosine of the CpG site on the top strand, and the 5'-end of said second MSPE primer comprises a second unique sequence that does not hybridize to any DNA sequences in the sample;
or wherein
(d) the primer extension reaction is performed using at least one pair of methylation specific primer extension (MSPE) primers that hybridize to the top strand of the amplified DNA, dNTPs, labeled ddCTP and a DNA polymerase, wherein the 3'-end of the first MSPE primer in the pair comprises a polynucleotide sequence that is specific for the top strand of the methylated DNA, the 3' terminal residue of the primer hybridizes at the cytosine residue of the CpG site to be analyzed, and the 5'-end of said first MSPE primer comprises a first unique sequence that does not hybridize to any DNA sequences in the sample; wherein the 3'-end of the second MSPE primer in the pair comprises a polynucleotide sequence that is specific for the top strand of the unmethylated DNA sequence, the 3' terminal residue of the primer hybridizes at the thymine residue which is derived from the cytosine of the CpG site to be analyzed, and the 5'-end of said second MSPE primer comprises a second unique sequence that does not hybridize to any DNA sequences in the sample;
or wherein
(d) the primer extension reaction is performed using at least one pair of methylation specific primer extension (MSPE) primers that hybridize to the top strand of the amplified DNA, mixture of labeled dNTPs and ddNTPs, mixture of unlabeled dNTPs and ddNTPs, and a DNA polymerase, wherein the 3'-end of the first MSPE primer in the pair comprises a polynucleotide sequence that is specific for the top strand of the methylated DNA, the 3' terminal residue of the primer hybridizes at the cytosine residue of the CpG site to be analyzed, and the 5'-end of said first MSPE primer comprises a first unique sequence that does not hybridize to any DNA sequences in the sample; wherein the 3'-end of the second MSPE primer in the pair comprises a polynucleotide sequence that is specific for the top strand of the unmethylated DNA sequence, the 3' terminal residue of the primer hybridizes at the thymine residue which is derived from the cytosine of the CpG site to be analyzed, and the 5'-end of said second MSPE primer comprises a second unique sequence that does not hybridize to any DNA sequences in the sample; or wherein
(d) the primer extension reaction is performed using at least one pair of MSPE primers that hybridize to the bottom strand of the amplified DNA, mixture of labeled dNTPs and ddNTPs, mixture of unlabeled dNTPs and ddNTPs, and a DNA polymerase, wherein the 3'-end of the first MSPE primer in the pair comprises a polynucleotide sequence that is specific for the bottom strand of the methylated DNA, the 3' terminal residue of the primer hybridizes at the guanine residue complementary to the cytosine of the CpG site on the top strand, and the 5'-end of said first MSPE primer comprises a first unique sequence that does not hybridize to any DNA sequences in the sample; wherein the 3'-end of the second MSPE primer in the pair comprises a polynucleotide sequence that is specific for the bottom strand of the unmethylated DNA sequence, the 3' terminal residue of the primer hybridizes at the adenine residue which is derived from the cytosine of the CpG site on the top strand, and the 5'-end of said second MSPE primer comprises a second unique sequence that does not hybridize to any DNA sequences in the sample;
or wherein
(d) the primer extension reaction is performed using at least a pair of MSPE primers that hybridize to the top strand of the amplified DNA, at least one labeled reverse primer, dNTPs, and a DNA polymerase, wherein the 5'-end of the first MSPE primer in the pair comprises a first unique sequence that does not hybridize to any DNA sequences in the sample, and the 3'-end of the first MSPE primer comprises a polynucleotide sequence that is specific for the top strand of the methylated DNA, the 3' terminal residue of the primer hybridizes at the cytosine residue of the CpG site to be analyzed; wherein the 5'-end of the second MSPE primer in the pair comprises a second unique sequence that does not hybridize to any DNA sequences in the sample, and the 3'-end of the second MSPE primer in the pair comprises a polynucleotide sequence that is specific for the top strand of the unmethylated DNA, the 3' terminal residue of the primer hybridizes at the thymine residue which is derived from the cytosine of the CpG site to be analyzed; and
(e) the primer extension products from (d) are hybridized to at least a pair of oligonucleotides, wherein the first oligonucleotide in the pair is the same as the first unique sequence and the second oligonucleotide in the pair is the same as the second unique sequence;
or wherein
(d) the primer extension reaction is performed using at least a pair of MSPE primers that hybridize to the bottom strand of the amplified DNA, at least one labeled reverse primer, dNTPs, and a DNA polymerase, wherein the 5'-end of the first MSPE primer in the pair comprises a first unique sequence that does not hybridize to any DNA sequences in the sample, and the 3'-end of the first MSPE primer comprises a polynucleotide sequence that is specific for the bottom strand of the methylated DNA, the 3' terminal residue of the primer hybridizes at the guanine residue complementary to the cytosine of the CpG site on the top strand; wherein the 5'-end of the second MSPE primer in the pair comprises a second unique sequence that does not hybridize to any DNA sequences in the sample, and the 3'-end of the second MSPE primer in the pair comprises a polynucleotide sequence that is specific for the bottom strand of the unmethylated DNA, the 3' terminal residue of the primer hybridizes at the adenine residue which is derived from the cytosine of the CpG site on the top strand; and
(e) the primer extension products from (d) are hybridized to at least a pair of oligonucleotides, wherein the first oligonucleotide in the pair is the same as the first unique sequence and the second oligonucleotide in the pair is the same as the second unique sequence.

2. Use of the method of claim 1 for detecting a disease or disorder in a subject, wherein a significant difference in the methylation status of a cytosine residue of a CpG site in a nucleic acid sequence from said subject, as determined using the method of claim 1, compared to the methylation status of the same CpG site in a nucleic acid sequence from a subject or standard not having the disease or disorder, as determined using the method of claim 1, is indicative of the presence of said disease or disorder in said subject.

3. A kit for the detection of methylated CpG-containing nucleic acid from a sample comprising: (1) a bisulfite compound; (2) primers for amplification of the nucleic acid molecule; (3) MSPE primers as defined in claim 1 for methylated CpG-containing nucleic acids; (4) MSPE primers as defined in claim 1 for unmethylated CpG-containing nucleic acids; (5) labeled and unlabeled dNTPs; (6) labeled and unlabeled ddNTPs; and (7) labeled or unlabeled reverse primers.

## Patentansprüche

1. Verfahren zum Nachweis einer 5'-Methylierung der DNA an einem Cytosin-Rest eines CpG-Bereichs in einer Nukleinsäure-Sequenz, umfassend:
(a) Gewinnen von DNA aus einer zu untersuchenden Probe;
(b) in Kontakt bringen der DNA mit einer Bisulfit-Verbindung;
(c) Amplifizieren der DNA unter Verwendung strang-spezifischer Primer;
(d) Durchführen einer Primerverlängerungs-Reaktion unter Verwendung von mindestens einem Paar von Primern, die für die methylierungs-spezifische Primerverlängerung (methylation specific primer extension; MSPE) vorgesehen sind und mit dem oberen Strang der amplifizierten DNA hybridisieren, sowie von markierten dNTPs und einer DNA-Polymerase, wobei das 3'-Ende des ersten MSPE-Primers in dem Paar eine Polynukleotid-Sequenz umfasst, die für den oberen Strang der methylierten DNA spezifisch ist, wobei der Rest am 3'-Ende des Primers mit dem Cytosin-Rest des zu untersuchenden CpG-Bereichs hybridisiert, und wobei das 5'-Ende des ersten MSPE-Primers eine erste einzigartige Sequenz umfasst, die nicht mit irgendwelchen DNA-Sequenzen in der Probe hybridisiert; wobei das 3'-Ende des zweiten MSPE-Primers in dem Paar eine Polynukleotid-Sequenz umfasst, die für den oberen Strang der nicht-methylierten DNA-Sequenz spezifisch ist, wobei der Rest am 3'-Ende des Primers mit dem Thymin-Rest hybridisiert, der aus dem Cytosin des zu untersuchenden CpG-Bereichs stammt, und wobei das 5'-Ende des zweiten MSPE-Primers eine zweite einzigartige Sequenz umfasst, die nicht mit irgendwelchen DNA-Sequenzen in der Probe hybridisiert;
(e) Hybridisieren der Primerverlängerungs-Produkte aus (d) mit mindestens einem Paar von Oligonukleotiden, wobei das erste Oligonukleotid in dem Paar komplementär zur ersten einzigartigen Sequenz ist, und das zweite Oligonukleotid in dem Paar komplementär zur zweiten einzigartigen Sequenz ist; und
(f) Bestimmen des 5'-Methylierungs-Zustands am Cytosin-Rest des CpG-Bereichs durch Vergleich der Hybridisierungs-Intensität der methylierten DNA mit der Hybridisierungs-Intensität der nicht-methylierten DNA in der Probe;
oder wobei
(d) die Primerverlängerungs-Reaktion unter Verwendung von mindestens einem Paar von MSPE-Primern durchgeführt wird, die mit dem unteren Strang der amplifizierten DNA hybridisieren, sowie von markierten dNTPs und einer DNA-Polymerase, wobei das 3'-Ende des ersten MSPE-Primers in dem Paar eine Polynukleotid-Sequenz umfasst, die für den unteren Strang der methylierten DNA spezifisch ist, wobei der Rest am 3'-Ende des Primers mit dem Guanin-Rest hybridisiert, der komplementär zu dem Cytosin des CpG-Bereichs auf dem oberen Strang ist, und wobei das 5'-Ende des ersten MSPE-Primers eine erste einzigartige Sequenz umfasst, die nicht mit irgendwelchen DNA-Sequenzen in der Probe hybridisiert; wobei das 3'-Ende des zweiten MSPE-Primers in dem Paar eine Polynukleotid-Sequenz umfasst, die für den unteren Strang der nicht-methylierten DNA-Sequenz spezifisch ist, wobei der Rest am 3'-Ende des Primers mit dem Adenin-Rest hybridisiert, der von dem Cytosin des CpG-Bereichs auf dem oberen Strang stammt, und wobei das 5'-Ende des zweiten MSPE-Primers eine zweite einzigartige Sequenz umfasst, die nicht mit irgendwelchen DNA-Sequenzen in der Probe hybridisiert;
oder wobei
(d) die Primerverlängerungs-Reaktion unter Verwendung von mindestens einem Paar von MSPE-Primern durchgeführt wird, die für die methylierungs-spezifische Primerverlängerung vorgesehen sind und mit dem oberen Strang der amplifizierten DNA hybridisieren, sowie von dNTPs, markiertem ddCTP und einer DNA-Polymerase, wobei das 3'-Ende des ersten MSPE-Primers in dem Paar eine Polynukleotid-Sequenz umfasst, die für den oberen Strang der methylierten DNA spezifisch ist, wobei der Rest am 3'-Ende des Primers mit dem Cytosin-Rest des zu untersuchenden CpG-Bereichs hybridisiert, und wobei das 5'-Ende des ersten MSPE-Primers eine erste einzigartige Sequenz umfasst, die nicht mit irgendwelchen DNA-Sequenzen in der Probe hybridisiert; wobei das 3'-Ende des zweiten MSPE-Primers in dem Paar eine Polynukleotid-Sequenz umfasst, die für den oberen Strang der nicht-methylierten DNA-Sequenz spezifisch ist, wobei der Rest am 3'-Ende des Primers mit dem Thymin-Rest hybridisiert, der von dem Cytosin des zu untersuchenden CpG-Bereichs stammt, und wobei das 5'-Ende des zweiten MSPE-Primers eine zweite einzigartige Sequenz umfasst, die nicht mit irgendwelchen DNA-Sequenzen in der Probe hybridisiert;
oder wobei
(d) die Primerverlängerungs-Reaktion unter Verwendung von mindestens einem Paar von MSPE-Primern durchgeführt wird, die für die methylierungs-spezifische Primerverlängerung vorgesehen sind und mit dem oberen Strang der amplifizierten DNA hybridisieren, sowie von einer Mischung von markierten dNTPs und ddNTPs, einer Mischung von unmarkierten dNTPs und ddNTPs, und einer DNA-Polymerase, wobei das 3'-Ende des ersten MSPE-Primers in dem Paar eine Polynukleotid-Sequenz umfasst, die für den oberen Strang der methylierten DNA spezifisch ist, wobei der Rest am 3'-Ende des Primers mit dem Cytosin-Rest des zu untersuchenden CpG-Bereichs hybridisiert, und wobei das 5'-Ende des ersten MSPE-Primers eine erste einzigartige Sequenz umfasst, die nicht mit irgendwelchen DNA-Sequenzen in der Probe hybridisiert; wobei das 3'-Ende des zweiten MSPE-Primers in dem Paar eine Polynukleotid-Sequenz umfasst, die für den oberen Strang der nicht-methylierten DNA-Sequenz spezifisch ist, wobei der Rest am 3'-Ende des Primers mit dem Thymin-Rest hybridisiert, der von dem Cytosin des zu untersuchenden CpG-Bereichs stammt, und wobei das 5'-Ende des zweiten MSPE-Primers eine zweite einzigartige Sequenz umfasst, die nicht mit irgendwelchen DNA-Sequenzen in der Probe hybridisiert;
oder wobei
(d) die Primerverlängerungs-Reaktion unter Verwendung von mindestens einem Paar von MSPE-Primern durchgeführt wird, die mit dem unteren Strang der amplifizierten DNA hybridisieren, sowie von einer Mischung von markierten dNTPs und ddNTPs, einer Mischung von unmarkierten dNTPs und ddNTPs, und einer DNA-Polymerase, wobei das 3'-Ende des ersten MSPE-Primers in dem Paar eine Polynukleotid-Sequenz umfasst, die für den unteren Strang der methylierten DNA spezifisch ist, wobei der Rest am 3'-Ende des Primers mit dem Guanin-Rest hybridisiert, der komplementär zu dem Cytosin des CpG-Bereichs auf dem oberen Strang ist, und wobei das 5'-Ende des ersten MSPE-Primers eine erste einzigartige Sequenz umfasst, die nicht mit irgendwelchen DNA-Sequenzen in der Probe hybridisiert; wobei das 3'-Ende des zweiten MSPE-Primers in dem Paar eine Polynukleotid-Sequenz umfasst, die für den unteren Strang der nicht-methylierten DNA-Sequenz spezifisch ist, wobei der Rest am 3'-Ende des Primers mit dem Adenin-Rest hybridisiert, der von dem Cytosin des CpG-Bereichs auf dem oberen Strang stammt, und wobei das 5'-Ende des zweiten MSPE-Primers eine zweite einzigartige Sequenz umfasst, die nicht mit irgendwelchen DNA-Sequenzen in der Probe hybridisiert;
oder wobei
(d) die Primerverlängerungs-Reaktion unter Verwendung von mindestens einem Paar von MSPE-Primern durchgeführt wird, die mit dem oberen Strang der amplifizierten DNA hybridisieren, sowie von mindestens einem markierten Reverse-Primer, dNTPs und einer DNA-Polymerase, wobei das 5'-Ende des ersten MSPE-Primers in dem Paar eine erste einzigartige Sequenz umfasst, die nicht mit irgendwelchen DNA-Sequenzen in der Probe hybridisiert, und wobei das 3'-Ende des ersten MSPE-Primers eine Polynukleotid-Sequenz umfasst, die für den oberen Strang der methylierten DNA-Sequenz spezifisch ist, wobei der Rest am 3'-Ende des Primers mit dem Cytosin-Rest des zu untersuchenden CpG-Bereichs hybridisiert; wobei das 5'-Ende des zweiten MSPE-Primers in dem Paar eine zweite einzigartige Sequenz umfasst, die nicht mit irgendwelchen DNA-Sequenzen in der Probe hybridisiert, und wobei das 3'-Ende des zweiten MSPE-Primers in dem Paar eine Polynukleotid-Sequenz umfasst, die für den oberen Strang der nicht-methylierten DNA spezifisch ist, und wobei der Rest am 3'-Ende des Primers mit dem Thymin-Rest hybridisiert, welcher von dem Cytosin des zu untersuchenden CpG-Bereichs stammt; und
(e) die Primerverlängerungs-Produkte aus (d) mit mindestens einem Paar von Oligonukleotiden hybridisiert werden, wobei das erste Oligonukleotid in dem Paar das gleiche ist wie die erste einzigartige Sequenz, und das zweite Oligonukleotid in dem Paar das gleiche ist wie die zweite einzigartige Sequenz;
oder wobei
(d) die Primerverlängerungs-Reaktion unter Verwendung von mindestens einem Paar von MSPE-Primern durchgeführt wird, die mit dem unteren Strang der amplifizierten DNA hybridisieren, sowie von mindestens einem markierten Reverse-Primer, dNTPs, und einer DNA-Polymerase, wobei das 5'-Ende des ersten MSPE-Primers in dem Paar eine erste einzigartige Sequenz umfasst, die nicht mit irgendwelchen DNA-Sequenzen in der Probe hybridisiert, und wobei das 3'-Ende des ersten MSPE-Primers eine Polynukleotid-Sequenz umfasst, die für den unteren Strang der methylierten DNA-Sequenz spezifisch ist, wobei der Rest am 3'-Ende des Primers mit dem Guanin-Rest hybridisiert, welcher komplementär zu dem Cytosin des CpG-Bereichs auf dem oberen Strang ist; wobei das 5'-Ende des zweiten MSPE-Primers in dem Paar eine zweite einzigartige Sequenz umfasst, die nicht mit irgendwelchen DNA-Sequenzen in der Probe hybridisiert, und wobei das 3'-Ende des zweiten MSPE-Primers in dem Paar eine Polynukleotid-Sequenz umfasst, die für den unteren Strang der nicht methylierten DNA spezifisch ist, und wobei der Rest am 3'-Ende des Primers mit dem Adenin-Rest hybridisiert, welcher von dem Cytosin des CpG-Bereichs auf dem oberen Strang stammt; und
(e) die Primerverlängerungs-Produkte aus (d) mit mindestens einem Paar von Oligonukleotiden hybridisiert werden, wobei das erste Oligonukleotid in dem Paar das gleiche ist wie die erste einzigartige Sequenz, und das zweite Oligonukleotid in dem Paar das gleiche ist wie die zweite einzigartige Sequenz.

2. Verwendung des Verfahrens nach Anspruch 1 zum Nachweis einer Krankheit oder einer Störung in einem Probanden, wobei ein signifikanter Unterschied im Methylierungszustand eines Cytosin-Restes eines CpG-Bereichs in einer Nukleinsäure-Sequenz von dem Probanden, wie er durch das Verfahren nach Anspruch 1 bestimmt wurde, verglichen mit dem Methylierungszustand desselben CpG-Bereichs in einer Nukleinsäure-Sequenz aus einem Probanden oder einem Standard, der die Krankheit oder Störung nicht aufweist, wie er durch das Verfahren nach Anspruch 1 bestimmt wurde, auf das Vorliegen dieser Krankheit oder Störung in dem Probanden hindeutet.

3. Kit für den Nachweis einer Nukleinsäure, welche methyliertes CpG enthält, aus einer Probe, wobei der Kit umfasst: (1) eine Bisulfit-Verbindung; (2) Primer für die Amplifikation des Nukleinsäure-Moleküls; (3) MSPE-Primer, wie in Anspruch 1 definiert für Nukleinsäuren, die methyliertes CpG enthalten; (4) MSPE-Primer, wie in Anspruch 1 definiert für Nukleinsäuren, die nicht-methyliertes CpG enthalten; (5) markierte und unmarkierte dNTPs; (6) markierte und unmarkierte ddNTPs; und (7) markierte oder unmarkierte Reverse-Primer.

## Revendications

1. Procédé de détection de la méthylation 5' de l'ADN sur un résidu cytosine d'un site CpG au sein d'une séquence d'acides nucléiques, comprenant :
(a) l'obtention d'ADN à partir d'un échantillon devant être analysé ;
(b) la mise en contact de l'ADN avec un composé de bisulfite ;
(c) l'amplification de l'ADN en utilisant des amorces spécifiques aux brins ;
(d) la réalisation d'une réaction d'extension d'amorces en utilisant au moins une paire d'amorces d'extension d'amorces spécifiques de la méthylation (MSPE) qui s'hybrident au brin supérieur de l'ADN amplifié, dNTPs marqués, et une ADN polymérase, dans lequel l'extrémité 3' de la première amorce MSPE dans la paire comprend une séquence polynucléotidique qui est spécifique du brin supérieur de l'ADN méthylé, le résidu terminal 3' de l'amorce s'hybride sur le résidu cytosine du site CpG devant être analysé, et l'extrémité 5' de ladite première amorce MSPE comprend une première séquence unique qui ne s'hybride à aucune séquence d'ADN dans l'échantillon ; dans lequel l'extrémité 3' de la seconde amorce MSPE dans la paire comprend une séquence polynucléotidique qui est spécifique du brin supérieur de la séquence d'ADN non méthylé, le résidu terminal 3' de l'amorce s'hybride sur le résidu thymine qui est dérivé de la cytosine du site CpG devant être analysé, et l'extrémité 5' de ladite seconde amorce MSPE comprend une seconde séquence unique qui ne s'hybride à aucune séquence d'ADN dans l'échantillon ;
(e) l'hybridation des produits de l'extension d'amorces à partir de (d) à au moins une paire d'oligonucléotides, dans lequel le premier oligonucléotide dans la paire est complémentaire de ladite première séquence unique, et le second oligonucléotide dans la paire est complémentaire de ladite seconde séquence unique ; et
(f) la détermination de l'état de la méthylation 5' sur le résidu cytosine du site CpG en comparant l'intensité d'hybridation de l'ADN méthylé à l'intensité d'hybridation de l'ADN non méthylé dans l'échantillon ;
ou dans lequel
(d) la réaction d'extension d'amorces est réalisée en utilisant au moins une paire d'amorces MSPE qui s'hybrident au brin inférieur de l'ADN amplifié, dNTPs marqués, et une ADN polymérase, dans lequel l'extrémité 3' de la première amorce MSPE dans la paire comprend une séquence polynucléotidique qui est spécifique du brin inférieur de l'ADN méthylé, le résidu terminal 3' de l'amorce s'hybride sur le résidu guanine complémentaire de la cytosine du site CpG sur le brin supérieur, et l'extrémité 5' de ladite première amorce MSPE comprend une première séquence unique qui ne s'hybride à aucune séquence d'ADN dans l'échantillon ; dans lequel l'extrémité 3' de la seconde amorce MSPE dans la paire comprend une séquence polynucléotidique qui est spécifique du brin inférieur de la séquence d'ADN non méthylé, le résidu terminal 3' de l'amorce s'hybride sur le résidu adénine qui est dérivé de la cytosine du site CpG sur le brin supérieur, et l'extrémité 5' de ladite seconde amorce MSPE comprend une seconde séquence unique qui ne s'hybride à aucune séquence d'ADN dans l'échantillon ;
ou dans lequel
(d) la réaction d'extension d'amorces est réalisée en utilisant au moins une paire d'amorces d'extension d'amorces spécifiques de la méthylation (MSPE) qui s'hybrident au brin supérieur de l'ADN amplifié, dNTPs, ddCTP marqué et une ADN polymérase, dans lequel l'extrémité 3' de la première amorce MSPE dans la paire comprend une séquence polynucléotidique qui est spécifique du brin supérieur de l'ADN méthylé, le résidu terminal 3' de l'amorce s'hybride sur le résidu cytosine du site CpG devant être analysé, et l'extrémité 5' de ladite première amorce MSPE comprend une première séquence unique qui ne s'hybride à aucune séquence d'ADN dans l'échantillon ; dans lequel l'extrémité 3' de la seconde amorce MSPE dans la paire comprend une séquence polynucléotidique qui est spécifique du brin supérieur de la séquence d'ADN non méthylé, le résidu terminal 3' de l'amorce s'hybride sur le résidu thymine qui est dérivé de la cytosine du site CpG devant être analysé, et l'extrémité 5' de ladite seconde amorce MSPE comprend une seconde séquence unique qui ne s'hybride à aucune séquence d'ADN dans l'échantillon ;
ou dans lequel
(d) la réaction d'extension d'amorces est réalisée en utilisant au moins une paire d'amorces d'extension d'amorces spécifiques de la méthylation (MSPE) qui s'hybrident au brin supérieur de l'ADN amplifié, mélange de dNTPs et ddNTPs marqués, mélange de dNTPs et ddNTPs non marqués, et une ADN polymérase, dans lequel l'extrémité 3' de la première amorce MSPE dans la paire comprend une séquence polynucléotidique qui est spécifique du brin supérieur de l'ADN méthylé, le résidu terminal 3' de l'amorce s'hybride sur le résidu cytosine du site CpG devant être analysé, et l'extrémité 5' de ladite première amorce MSPE comprend une première séquence unique qui ne s'hybride à aucune séquence d'ADN dans l'échantillon ; dans lequel l'extrémité 3' de la seconde amorce MSPE dans la paire comprend une séquence polynucléotidique qui est spécifique du brin supérieur de la séquence d'ADN non méthylé, le résidu terminal 3' de l'amorce s'hybride sur le résidu thymine qui est dérivé de la cytosine du site CpG devant être analysé, et l'extrémité 5' de ladite seconde amorce MSPE comprend une seconde séquence unique qui ne s'hybride à aucune séquence d'ADN dans l'échantillon ;
ou dans lequel
(d) la réaction d'extension d'amorces est réalisée en utilisant au moins une paire d'amorces MSPE qui s'hybrident au brin inférieur de l'ADN amplifié, mélange de dNTPs et ddNTPs marqués, mélange de dNTPs et ddNTPs non marqués, et une ADN polymérase, dans lequel l'extrémité 3' de la première amorce MSPE dans la paire comprend une séquence polynucléotidique qui est spécifique du brin inférieur de l'ADN méthylé, le résidu terminal 3' de l'amorce s'hybride sur le résidu guanine complémentaire de la cytosine du site CpG sur le brin supérieur, et l'extrémité 5' de ladite première amorce MSPE comprend une première séquence unique qui ne s'hybride à aucune séquence d'ADN dans l'échantillon ; dans lequel l'extrémité 3' de la seconde amorce MSPE dans la paire comprend une séquence polynucléotidique qui est spécifique du brin inférieur de la séquence d'ADN non méthylé, le résidu terminal 3' de l'amorce s'hybride sur le résidu adénine qui est dérivé de la cytosine du site CpG sur le brin supérieur, et l'extrémité 5' de ladite seconde amorce MSPE comprend une seconde séquence unique qui ne s'hybride à aucune séquence d'ADN dans l'échantillon ;
ou dans lequel
(d) la réaction d'extension d'amorces est réalisée en utilisant au moins une paire d'amorces MSPE qui s'hybrident au brin supérieur de l'ADN amplifié, au moins une amorce reverse marquée, dNTPs, et une ADN polymérase, dans lequel l'extrémité 5' de la première amorce MSPE dans la paire comprend une première séquence unique qui ne s'hybride à aucune séquence d'ADN dans l'échantillon, et l'extrémité 3' de la première amorce MSPE comprend une séquence polynucléotidique qui est spécifique du brin supérieur de l'ADN méthylé, le résidu terminal 3' de l'amorce s'hybride sur le résidu cytosine du site CpG devant être analysé ; dans lequel l'extrémité 5' de la seconde amorce MSPE dans la paire comprend une seconde séquence unique qui ne s'hybride à aucune séquence d'ADN dans l'échantillon, et l'extrémité 3' de la seconde amorce MSPE dans la paire comprend une séquence polynucléotidique qui est spécifique du brin supérieur de l'ADN non méthylé, le résidu terminal 3' de l'amorce s'hybride sur le résidu thymine qui est dérivé de la cytosine du site CpG devant être analysé ; et
(e) les produits de l'extension d'amorces à partir de (d) sont hybridés à au moins une paire d'oligonucléotides, dans lequel le premier oligonucléotide dans la paire est le même que la première séquence unique et le second oligonucléotide dans la paire est le même que la seconde séquence unique ;
ou dans lequel
(d) la réaction d'extension d'amorces est réalisée en utilisant au moins une paire d'amorces MSPE qui s'hybrident au brin inférieur de l'ADN amplifié, au moins une amorce reverse marquée, dNTPs, et une ADN polymérase, dans lequel l'extrémité 5' de la première amorce MSPE dans la paire comprend une première séquence unique qui ne s'hybride à aucune séquence d'ADN dans l'échantillon, et l'extrémité 3' de la première amorce MSPE comprend une séquence polynucléotidique qui est spécifique du brin inférieur de l'ADN méthylé, le résidu terminal 3' de l'amorce s'hybride sur le résidu guanine complémentaire de la cytosine du site CpG sur le brin supérieur ; dans lequel l'extrémité 5' de la seconde amorce MSPE dans la paire comprend une seconde séquence unique qui ne s'hybride à aucune séquence d'ADN dans l'échantillon, et l'extrémité 3' de la seconde amorce MSPE dans la paire comprend une séquence polynucléotidique qui est spécifique du brin inférieur de l'ADN non méthylé, le résidu terminal 3' de l'amorce s'hybride sur le résidu adénine qui est dérivé de la cytosine du site CpG sur le brin supérieur ; et
e) les produits de l'extension d'amorces à partir de (d) sont hybridés à au moins une paire d'oligonucléotides, dans lequel le premier oligonucléotide dans la paire est le même que la première séquence unique et le second oligonucléotide dans la paire est le même que la seconde séquence unique.

2. Utilisation du procédé selon la revendication 1 pour la détection d'une maladie ou d'un trouble chez un sujet, dans lequel une différence importante de l'état de méthylation d'un résidu de cytosine d'un site CpG dans une séquence d'acides nucléiques dudit sujet, telle que déterminée en utilisant le procédé de la revendication 1, par comparaison à l'état de méthylation du même site CpG dans une séquence d'acides nucléiques d'un sujet ou standard n'ayant pas la maladie ou le trouble, telle que déterminée en utilisant le procédé de la revendication 1, indique la présence de ladite maladie ou dudit trouble chez ledit sujet.

3. Kit pour la détection d'acide nucléique contenant un CpG méthylé à partir d'un échantillon comprenant : (1) un composé de bisulfite ; (2) des amorces pour l'amplification de la molécule d'acides nucléiques ; (3) des amorces MSPE telles que définies dans la revendication 1 pour les acides nucléiques contenant un CpG méthylé ; (4) des amorces MSPE telles que définies dans la revendication 1 pour les acides nucléiques contenant un CpG non méthylé ; (5) des dNTPs marqués et non marqués ; (6) des ddNTPs marqués et non marqués ; et (7) des amorces reverses marquées et non marquées.
